# EUROPEAN PATENT SPECIFICATION

(11) **EP 0 469 144 B1**
(45) Date of publication and mention of the grant of the patent: **10.04.1996**
(21) Application number: 91906095.4
(22) Date of filing: 08.02.1991
(51) Int. Cl.: A23D 7/00

(54) **SAFFLOWER OIL**
SAFFLOROEL
HUILE DE CARTHAME

(30) Priority: 09.02.1990 US 478077; 09.02.1990 US 478079
(43) Date of publication of application: 05.02.1992
(60) Divisional application: 94201765.8
(73) Proprietor: THE RESEARCH AND DEVELOPMENT INSTITUTE, INC, Bozeman, MT 59714 (US)
(72) Inventor: BERGMAN, Jerald W., Sidney, MT 59270 (US)
(74) Representative: Crisp, David Norman
(86) International application number: US9100917
(87) International publication number: WO9111906

(56) References cited:
- EP-A- 0 044 723
- EP-A- 0 323 753
- FR-A- 2 617 675
- US-A- 3 186 854
- US-A- 3 529 974
- US-A- 4 627 192
- R.W.ALLARD 'Principles of plant breeding', 1960, John Wiley & Sons, New York, US, "Transgressive segregation", see page 472, lines 28-29; page 67, paragraph 2 - page 72, paragraph 1
- PLANTBREEDING, vol. 98, 1987, Berlin, DE, & N.N. Roy et al.: "Prospects for the development of rapeseed (B.napus L.) with improved linoleic and linolenic acid content ", see page 89, right-hand column, line 20 - page 91, left-hand column, line 4
- KIRK-OTHMER 'Encyclopedia of chemical technology, volume 9, 1978, John Wiley & Sons, New York, US, chapter "Fats and fatty oils", pages 795-831, see page 798, table 1; page 804 - page 805, table 3; page 812, paragraph 5; page 816, paragraph 4 - page 818, paragraph 2
- JOURN. AMERICAN OIL CHEMISTS SOC. vol. 49, no. 1, January 1972, pages 27 - 29; P.F.KNOWLES: 'The plant genetist's contribution towards changing lipid and amino acid composition of safflower ', see page 27, right-hand column, line 3 - page 29, right-hand column, line 5; tables 2,3 (cited in the application)
- H. KUCKUCK ET AL. 'Grundzüge der Pflanzenzüchtung'1985, Walther de Gruyter, Berlin, DE, paragraph 1.2 "Kombinationszüchtung pages 20 - 49 see page 21, line 1 - page 40, line 10

## Description

The present invention relates to safflower oil.

Safflower, Carthamus tinctorius L., is a highly branched, herbaceous, thistle-like annual varying in height from 30 to 150 cm. Safflower has been grown for centuries in small plots over a vast area from China to the Mediterranean region, and along the Nile Valley as far as Ethiopia. Since 1945 commercial production has taken place in the United States, Russia, Australia and Mexico. Safflower has remained a minor oilseed in terms of total production and world trade. The crop can be grown under full mechanization so that the production costs compare favorably with other oilseeds. In addition, safflower is suitable for growth in drier regions and thus provides a profitable alternative in such areas. Altering the oil content, increasing protein content or increasing seed yield are current objectives in safflower breeding programs.

Safflower oil is comprised primarily of palmitic, stearic, oleic and linoleic acids, with the vast majority comprised of oleic and linoleic acids. Two different types of oil can be produced, depending on the genetics of the particular safflower line. One type is high in linoleic acid and the other is high in oleic acid. A third experimental type of safflower oil contains about equal amounts of oleic and linoleic acids. A high oleic acid concentration of safflower oil is desirable when its end use is in the food industry. A high linoleic acid safflower oil is desirable when its end use is in resin and paint formulations. In all instances, it is desirable to produce a safflower oil which is low in the saturated fatty acids, i.e., palmitic and stearic acids.

Three allelic genes, Ol, ol' and ol, are the primary genetic determinants of the proportions of oleic and linoleic acids. The genotype olol has 72-80% oleic acid in the oil, and the genotype OlOl has 72-80% linoleic acid. The genotype ol'ol' has about 45% of each fatty acid. It has been discovered that additional minor genes may be involved in determining the fatty acid content of a particular safflower variety. Knowles, P.F., J. Am. Oil Chem. Soc. 49, 27 (1972) noted that such modifying genes may make it possible to raise the oleic acid content above 80%, but probably not above 85%.

Knowles, supra, further noted that although high oleic and high linoleic safflower varieties are temperature stable, there is a slight variation in fatty acid content depending on the growth temperature of the variety. This variation is illustrated in Table 1.

**TABLE 1**

| Fatty Acid Composition of Safflower Types Grown Under Different Temperatures | | | | |
|---|---|---|---|---|
| Oil Type | Temp.¹ | Sat.² | Fatty Acid (%) | |
| | | | Oleic | Linoleic |
| high linoleic | Low | 8.4 | 9.1 | 82.4 |
| | Int. | 7.8 | 14.0 | 78.2 |
| | High | 9.3 | 15.2 | 75.5 |
| high oleic | Low | 6.3 | 69.7 | 24.0 |
| | Int. | 7.0 | 71.6 | 21.4 |
| | High | 8.3 | 77.4 | 14.3 |

| | | | | |
|---|---|---|---|---|
| ¹ Day and night temperatures, respectively: low = 18.4°C and 15.6°C; intermediate = 26.7°C and 23.9°C; and high = 29.4°C and 26.1°C. | | | | |
| ² Saturates = palmitic and stearic acids. | | | | |

Thus, a high oleic acid safflower variety grown in Montana, for example, having a mean growing temperature of about 70°F, would be expected to have a slightly lower production of oleic acid than the same variety grown in California. Conversely, the variety grown in California would be expected to have a higher level of oleic acid than the value reported for the variety in Montana.

US-A-4627192 describes a sunflower seed and sunflower oil having a high oleic acid content (approximately 80% or greater) and a low linoleic acid content.

As previously mentioned, the present invention relates to a safflower oil.

More specifically one aspect of the present invention relates to a safflower oil comprising fatty acid, having a oleic acid content of greater than 80% up to 90% relative to the total fatty acid content of said oil, and having an AOM value of greater than 60 hours.

The oil may be conveniently obtained from safflower seed having an oleic acid content about 80% or greater relative to the total fatty acid content of the seed when the seed is produced at a mean temperature of about 70°F (21.1°C).

The oil may also be obtained from a safflower plant which produces seeds having the above described content of oleic acid when the plants are grown in an environment in which the mean temperature during seed development is about 70°F (21.1°C).

We have also found it possible to provide:
A Carthamus tinctorius L. seed product consisting of a substantially homogenous assemblage of safflower seeds having an oleic acid content of about 80% or greater, relative to the total fatty acid content of said seeds when said seeds are produced at a mean temperature of about 70°F (21.1°C).

A safflower seed having an oleic acid content of approximately 80% or greater, relative to the total fatty acid content of said seed, and a ratio of the amount of saturated fatty acid in said seed to the amount of oleic acid in said seed of less than about 0.08.

A safflower seed having an oleic acid content of about 80% to about 90% relative to the total fatty acid content of said seed and wherein the ratio of saturated fatty acids to the oleic acid is less than about 0.08.

A safflower plant having an oleic acid content of approximately 80% or greater, relative to the total fatty acid content of said seed, and a ratio of the amount of saturated fatty acid in said seed to the amount of oleic acid in said seed of less than about 0.08.

A safflower plant having an oleic acid content of about 80% to about 90% relative to the total fatty acid content of said seed and wherein the ratio of saturated fatty acids to the oleic acid is less than about 0.08.

A safflower seed having an oleic acid content of approximately 80% or greater, relative to the total fatty acid content of said seed, wherein said seed is a product of a cross between (A) a first parent from a safflower line which is true-breeding for said oleic acid content and (B) a parent from a second safflower line.

An individual safflower plant wherein the seeds therefrom have an oleic acid content of approximately 80% or greater, relative to the total fatty acid content of said seeds, wherein said plant is a product of a cross between (A) a first parent from a safflower line which is true-breeding for said oleic acid content and (B) a parent from a second safflower line.

A safflower oil produced by the process comprising extracting oil from a substantially homogeneous assemblage of safflower seeds, said safflower oil having an AOM value of greater than about 60 hours.

A safflower oil having an oleic acid content of about 80% or greater, relative to the total fatty acid content of the oil wherein the oil is substantially free of free fatty acids.

A bleached safflower oil having an oleic acid content of about 80% or greater, relative to the total fatty acid content of the oil.

A deodorized safflower oil having an oleic acid content of 80% or greater, relative to the total fatty acid content of said oil.

A safflower oil having an oleic acid content of 80% or greater, relative to the total fatty acid content of said oil, wherein the oil is deodorized, bleached and is substantially free of free fatty acids.

Preferably, the oleic acid content of the safflower oil in accordance with the present invention is between about 80% and about 84.7%, preferably, about 81.9% and about 85.9%, and even more preferably between about 83.4% and about 86.1%.

Preferably the safflower seed or plant used to prepare the oil in accordance with the present invention has an oleic acid content from about 81.9% to about 90%; a ratio of saturated fatty acids to oleic acid of less than about 0.07, preferably about 0.06.

Various preferred features and embodiments of the present invention will now be described by way of non-limiting example.

In order to provide an understanding of several of the terms used in the specification and claims, the following definitions are provided.

Homogeneous assemblage - The term homogeneous assemblage is intended to refer a group of seeds which are homogeneous for a given characteristic. This term is intended to include any seeds which are homogeneous as determined by any 1000 seeds from one acre of safflower.

Mean temperature - The mean temperature is the mean of the recorded daily temperatures during a specified period, such as a month or a developmental stage of safflower growth. The mean temperature (F) for the months of May through October from 1949 through the 1988-89 crop year is shown in Table 2.

**TABLE 2**

| Mean Temperatures (°F) for 1949-89 at the Eastern Agricultural Research Center, Sidney Montana | | | | | |
|---|---|---|---|---|---|
| Month | Mean Temp. | | Month | Mean Temp. | |
| | °F | (°C) | | °F | (°C) |
| May | 55.5 | (13.0) | Aug. | 67.8 | (19.9) |
| June | 64.2 | (17.9) | Sept. | 56.4 | (13.6) |
| July | 69.7 | (20.9) | Oct. | 45.6 | ( 7.6) |

Saturated Fatty acids - As used herein, the term saturated fatty acids is intended to include only palmitic acid and stearic acid.

Seed development or production - For the purposes of this invention, the seed development, seed production or production of seed is the time during safflower growth from pollination through seed maturation. In Montana, this time is usually from about the third week of July through the middle of August.

Series of crosses - A series of crosses is used herein to refer to the breeding technique of transgressive segregation in which multiple, single crosses are made to produce female and male parents.

As noted above, the content of various fatty acids such as oleic and linoleic, which is characteristic of oil from a given safflower seed sample, is commonly expressed as a percentage of the total fatty acid fraction in the oil. This convention will be followed in the description given below unless otherwise indicated. Dimensionless ratios of saturated fatty acid content to either oleic or linoleic acid content, which are also mentioned below, are calculated by dividing the saturated fatty acid percentage by either the oleic or the linoleic acid percentage, as applicable. As also noted above, the specific level of oleic acid, linoleic acid and saturated fatty acids is dependent upon the temperature during seed development. Unless indicated otherwise, the percentages shown for the contents of these fatty acids are for safflower grown in Montana and at a mean temperature of about 70°F (21.1°C) for seed development or production. As used herein, about 70°F (21.1°C) is intended to include mean temperatures of 70°F (21.1°C), plus or minus 2°F (0.6°C).

The present invention has a low level of saturated fatty acid in the oil. The fatty acid content in a ratio to the oleic acid is expressed as the combined weight of the two saturated fatty acids divided by the weight of the oleic acid. The measurement of the ratio is done using the extracted fatty acids, however, the oils may also be used because there is little difference in the methods and little difference in the weight of the oil versus the fatty acids.

The ratios for the saturated fatty acids to the oleic acid are thus preferably less than about 0.08, more preferably less than about 0.07, yet more preferably less than about 0.06.

For purposes of the present description, the terms "variety", "cultivar" and "line" are used synonymously to refer to a group of plants which are uniform in their traits such that there is relatively minor variation within the group and such variation can be characterized. The decreased variation within this group has generally (although not exclusively) resulted from several generations of self-pollination or selfing. A variety is considered true breeding for a particular trait if it is genetically homozygous for that trait to the extent that when the variety is self-pollinated, a significant amount of independent segregation of the trait among progeny is not observed.

Safflower is primarily self-pollinating, with some cross-pollination possible through the interaction of insects, primarily bees. Safflower pollen is too heavy for wind-aided cross-pollination to occur. Consequently, in arid areas, such as in Montana, there is little cross-pollination that occurs because of a low bee population. Safflower breeding, as described herein, is performed using conventional techniques to develop the high oleic acid and high linoleic acid varieties described and claimed.

The safflower of the present invention reproducibly expresses a high oleic acid trait,
and conversely, a low saturated fatty acid trait, against a phenotypic background of agronomical traits which is sufficiently consistent for commercial applications. In contrast, the original breeding material produced seeds having a lower oleic acid content,
and conversely, a higher saturated fatty acid content against a similar phenotypic background of agronomic traits. In accordance with one embodiment of the present invention, safflower seed is produced which contains in excess of 80%, preferably in excess of 81.9%, and most preferably in excess of 83.4%, up to 90%, oleic acid when produced at a mean seed development temperature of about 70°F.

To produce the safflower varieties used in the present invention, an extensive breeding program is used in which many parental varieties are utilized to pursue transgressive segregation. In order to accumulate all minor genes for various traits, it is necessary to keep the selection pressure low. In this breeding program varieties having the major ol gene (for high oleic)
are utilized and many crosses are made to accumulate minor, favorable genes into one genotype. Any genotype having desirable characteristics may be utilized in this breeding program. The intermediate lines which are produced are not crossed with any of the original breeding material. The genotypes are then selfed and selected for the specific level of oleic acid desired.

This general breeding procedure has given attention to the modifications in fatty acid composition and other desirable traits produced by genes having small effects on such traits. Since 1973, cultivars have been crossed and intercrossed on an annual basis to permit selection for transgressive segregation of superior lines for the desired traits, including oil quality, Alternaria leaf spot resistance, Pseudomonas bacterial blight resistance, oil content, drought resistance and other quantitative traits. Each year, selections have been made for traits or characteristics that are expressed. Approximately the top 50% of the breeding material is saved from each cycle of selection for intercrossing for the next cycle of selection and intercrossing. In this manner genes with small effects for desirable traits such as fatty acid composition, disease resistance, drought tolerance, etc., are incorporated into the continually improving germplasm.

Newly developed cultivars with the additional favorable genes are incorporated into the germplasm base by intercrossing with superior breeding lines. During the period from 1973 to 1982, selection was practiced to improve seed oil content, Alternaria leaf spot resistance, Pseudomonas bacterial blight resistance, Sclerotina head rot resistance, posthoused seed dormancy, drought resistance and other expressed and desirable traits. For example, seed oil content of the breeding material was improved approximately 1% each year, from 33% to 42% during the period from 1974 to 1982. Selection for improved fatty acid composition, either oleic acid content or linoleic acid content, began in 1985. Selection for transgressive segregation of superior lines for fatty acid composition has been practiced since 1985. Oil types have been obtained which are lower in saturated fatty acids with, corresponding higher levels of oleic acid or linoleic acid than previously reported.

The top 302 lines for oleic acid productions were selected from a group of 6000 crosses which were made in 1985. The 1985 lines produced from about 70.9% to 80.8% oleic acid in the seed. The linoleic acid content ranged from 12.4% to 22.1% and the saturated fatty acid content ranged from 4.7% to 6.8%. The ratio of saturated fatty acids to oleic acid ranged from 0.059 to 0.088, with two lines at 0.093 and 0.095. Only one of the 302 lines was above 80%.

These 302 lines were selfed in 1986 and the seed analyzed for oleic acid production. A total of 215 lines were selected after the 1986 season which produced seeds having an oleic acid content greater than 80%. The range in oleic acid content of these lines was 80% to 84.7%. The linoleic acid content ranged from 8.4% to 13.6% and the saturated fatty acid content ranged from 3.9% to 7.5%. The ratio of saturated fatty acids to oleic acid ranged from 0.047 to 0.086, with one line at 0.093. Twenty-four of these lines produced seeds having an oleic acid content of greater than 83%.

These 215 lines were selfed in 1987 and the seed analyzed for oleic acid production. A total of 303 lines were selected after the 1987 season which produced seeds having oleic acid content greater than 80%. The range in oleic acid content of these lines was 81.9% to 85.9%. The linoleic acid content ranged from 7.2% to 11.8% and the saturated fatty acid content ranged from 3.7% to 6.2%. The ratio of saturated fatty acids to oleic acid ranged from 0.044 to 0.076. Forty-eight of these lines produced seeds having an oleic acid content of greater than 84%.

These 303 lines were selfed in 1988 and the seed analyzed for oleic acid production. A total of 352 lines were selected after the 1988 season which produced seeds having oleic acid content greater than 80%. The range in oleic acid content of these lines was 83.4% to 86.1%. The linoleic acid content ranged from 7.4% to 10.4% and the saturated fatty acid content ranged from 4.7% to 5.8%, with six lines up to 6.5%. The ratio of saturated fatty acids to oleic acid ranged from 0.055 to 0.070, with three lines up to 0.075. Twenty-two of these lines produced seeds having an oleic acid content of greater than 85%.

These 352 lines were selfed in 1989 and the seed analyzed for oleic acid production. Slight changes were seen in the oleic acid content of the progeny, with some lines approaching 87%. In order to increase the oleic acid content to greater than 87%, and up to 90%, the previously obtained lines are intercrossed to derive new lines. These new lines produce seed having an oleic acid content of up to 90%.

The safflower varieties used in the present invention share characteristics common among commercial safflower varieties. Several of these characteristics will be set forth in more detail below.

### EXAMPLES

The following examples are provided to further illustrate the present invention and are non-limiting.

### EXAMPLE 1

### Determination of Fatty Acid Content

The fatty acid composition of safflower seeds developed in the breeding program was determined by gas-liquid chromatography (GLC) in accordance with the procedures described in Hougen, F.W. and Bodo, V., J. Am. Oil Chem. Soc. 50, 230 (1973) and Daun, J.K. and Mazur, P.B., J. Am. Oil Chem. Soc. 60, 1751 (1983); and, with techniques developed by D.I. McGregor, Agriculture Canada Research Station, Saskatoon, Saskatchewan, Canada for rapeseed and mustard.

### A) Analysis of 12 Gram Samples

A 12 gram sample of the safflower seed of interest was dried at 45-50°C for at least 4 hours. The sample was then ground in the presence of 40 ml of hexane using a tissue homogenizer for approximately 45-60 seconds. The container of the homogenate was then capped and allowed to settle for approximately 15 minutes. A 0.5 ml aliquot was then pipetted in a 13x100 mm glass test tube. To this tube was added 0.5 ml of benzene followed by 2 ml of sodium methoxide in methanol at a concentration of 2 g sodium methoxide in 100 ml methanol. The tube was capped and allowed to stand for 10 minutes. After standing, 3 drops of Bromophenol Blue in ethanol was added followed by drops of 1N HCl until the blue solution turned yellow. The excess HCl was then neutralized with 1N sodium carbonate until one drop turned the solution blue. Deionized water was then added to make sure separation of the two phases was complete. Approximately 1 ml of the upper phase was then placed in a septum sealed vial and analyzed by GLC.

### B) Analysis of 1 to 5 Seed Samples

The individual seeds were cut in half with a razor blade. The halves were then placed in a glass test tube with a small amount of hexane, approximately 1-2 ml. The pieces were then mashed and stirred for around 30 seconds. with a glass rod and allowed to sit with the tube capped for 15 minutes. A 0.5 ml sample of the hexane extractant was then treated in the manner described above. The fatty acid analysis of half a seed enabled the planting of the remaining half for further breeding.

### C) Gas-Liquid Chromatography

The GLC analyses were accomplished using a 5890 Hewlett-Packard instrument equipped with a flame ionization detector and a Hewlett-Packard 3392 integrator. The column used was a J&W Scientific fused silica capillary column coated with DB-225+ (film thickness of 0.25 microns, column dimensions of 15 meters x 0.318 mm, catalog no. 1232212). The operating conditions of the GLC included an injector temperature of 225°C and a detector temperature of 300°C. Column flow was 1.6 cc/min. of helium. Each chromatographic run was temperature programmed to begin at 170°C and remain at that temperature for 2.0 minutes. The temperature was then increased to 190°C at a rate of 3°C/min. The temperature was then held at 190°C for 1.5 minutes. The column temperature was increased to 220°C at a rate of 2°C/min. The temperature was held at 220°C for 3 minutes. After this period of time, the chromatograph was completed and the column prepared for the next run.

### Example 2

### Characterization of Parent Lines

The following is a description of several of the parent safflower lines utilized in the breeding program. Any parent material could be utilized as long as it met the criteria described above, namely the presence of desirable traits such as oil content, blight resistance and the like. These parent materials are readily available from public institutions or for sale by commercial organizations.

'Frio' safflower developed by the Arizona Agricultural Experiment Station (AES) and the Agricultural Research Service - U.S. Department of Agriculture (ARS-USDA) was released in 1965 and has the genotype OlOl that produces seed high in linoleic acid.

'Sidney Selection 87-14-6'is a 1965 selection for Alternaria leaf spot resistance made from the 1964 bulk composite of 555 safflower introductions of the 1960 world safflower collection.

'79AZ9543-1' is an experimental white flowered oleic safflower line obtained in 1980 from the Arizona AES.

'S-304' was developed by Seedtec International, Woodland California and has the genotype ol'ol' that produces an oil with approximately equal amount of linoleic and oleic acids, but the relative amounts of each fatty acid are greatly influenced by growing temperature.

'S-208' was developed by Seedtec International and is a normal white hull seed variety and has the genotype OlOl that produces seed high in linoleic acid. A white flowered individual plant from S-208 was used as one of the parental lines.

'UC-1' was developed in 1966 by the California AES and was the first commercial oleic safflower cultivar having the genotype olol that produces seed higher in oleic acid content.

'Oleic Leed' is a high oleic acid content cultivar released in 1974 by the ARS-USDA and the California AES. The oleic acid olol gene was obtained from 'UC-1' crossed to 'Leed' and backcrossed to 'Leed' two times.

'Th-5' is a parental line developed by the ARS-USDA and the Utah AES in 1970 and carries the thth gene pair which governs reduced pericarp. The line was released for use as a female parent in hybrid development programs.

'Carthamus nitidus' is a related species to C. tinctorius L. with 12 chromosome pairs.

'ol41-1' is an individual plant selection with extremely reduced hull or partial hull, with an iodine value of 141 obtained from A.L. Urie in 1974. This plant was obtained from a line segregating for linoleic gene Ol and oleic gene ol.

'US-10' was developed by the ARS-USDA and the California AES in 1959 and has the genotype OlOl that produces seed high in linoleic acid. A white flowered individual plant from 'US-10' was used as one of the parental lines.

Examples 3-9 are directed to the development of high oleic acid varieties.

### Example 3

### 1985 Breeding Program and Results

The top 302 lines for oleic acid production were selected from a group of 6000 crosses which were made in 1985. The 1985 lines produced from about 70.9% to 80.8% oleic acid in the seed. The linoleic acid content ranged from 12.4% to 22.1% and the saturated fatty acid content ranged from 4.7% to 6.8%. The ratio of saturated fatty acids to oleic acid ranged from 0.059 to 0.088, with two lines at 0.093 and 0.095. Only one of the 302 lines was above 80%.

The mean temperatures for July and August were 71.4°F (21.9°F) and 66.3°F (19.1°C) respectively. The results are shown in Table 3.

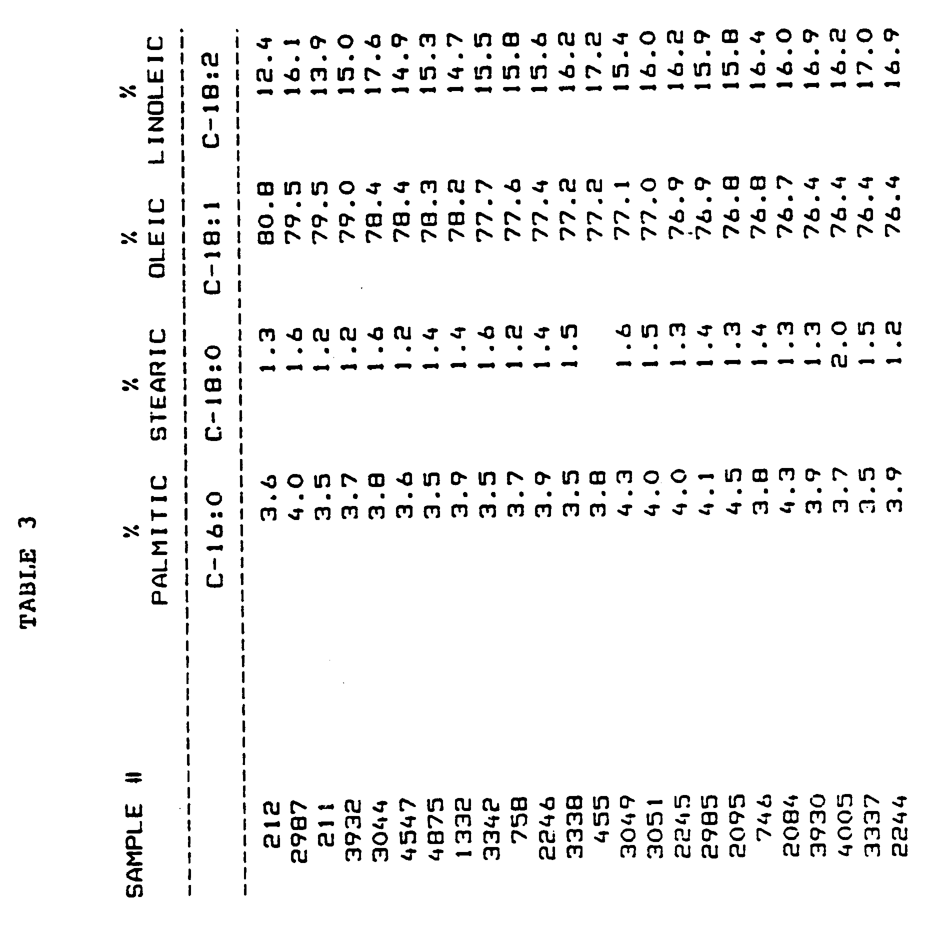

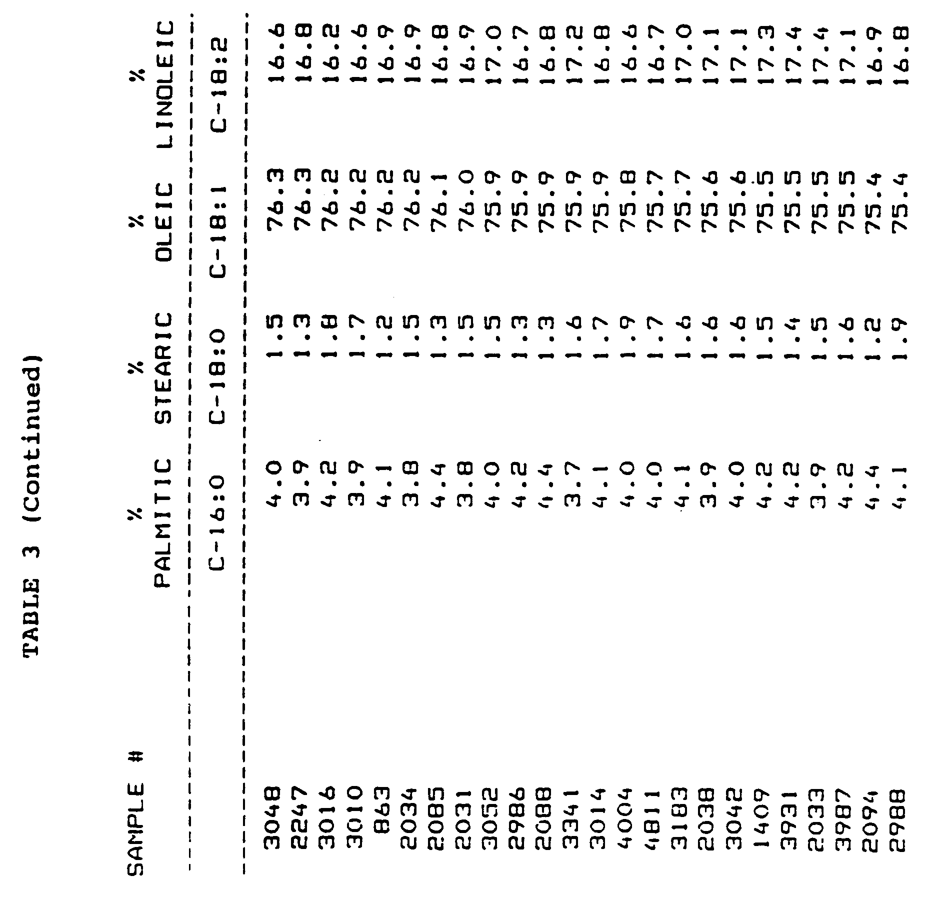

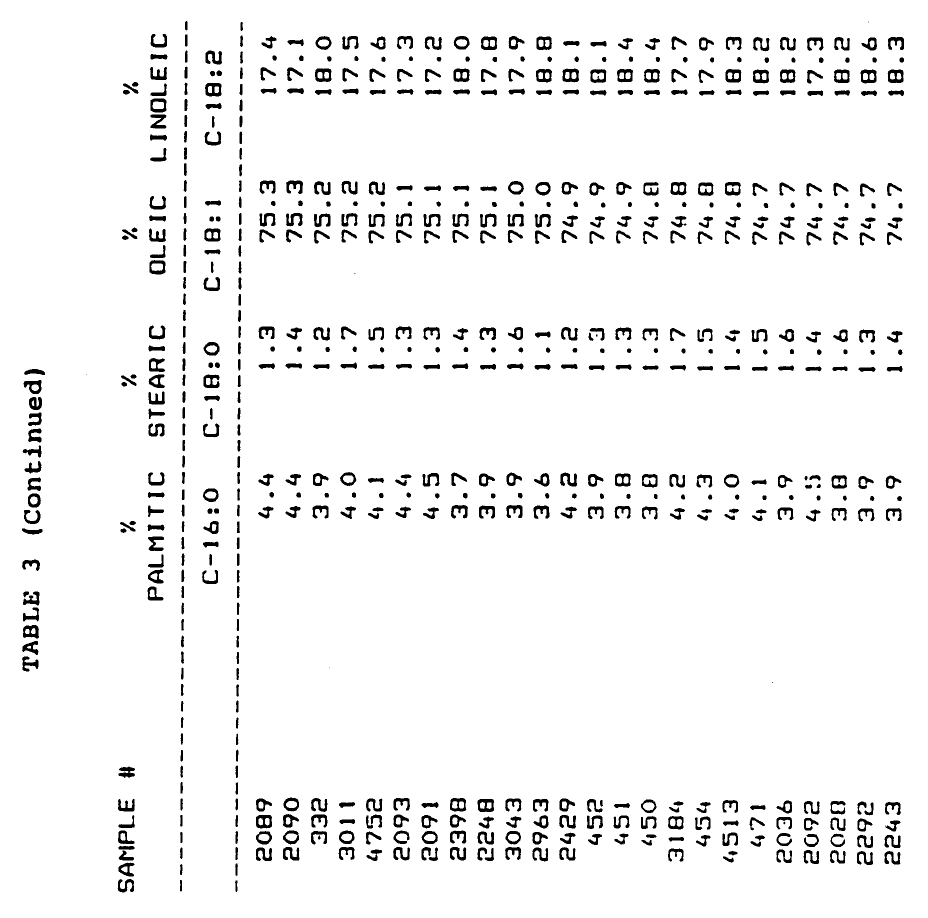

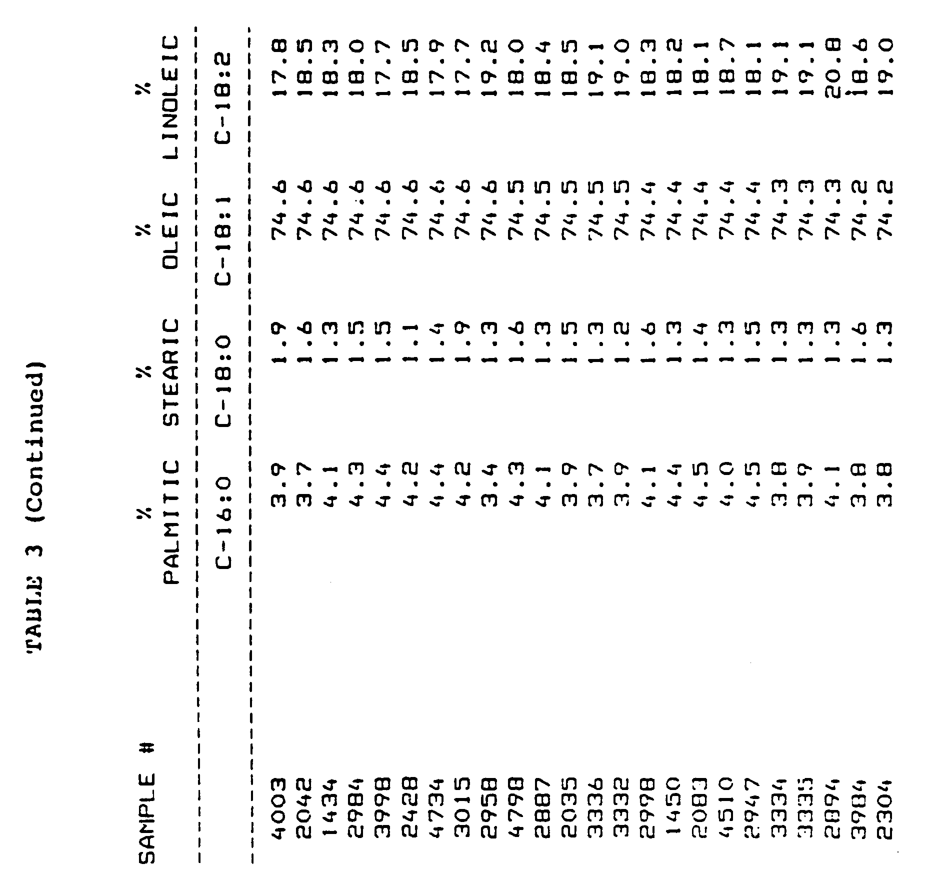

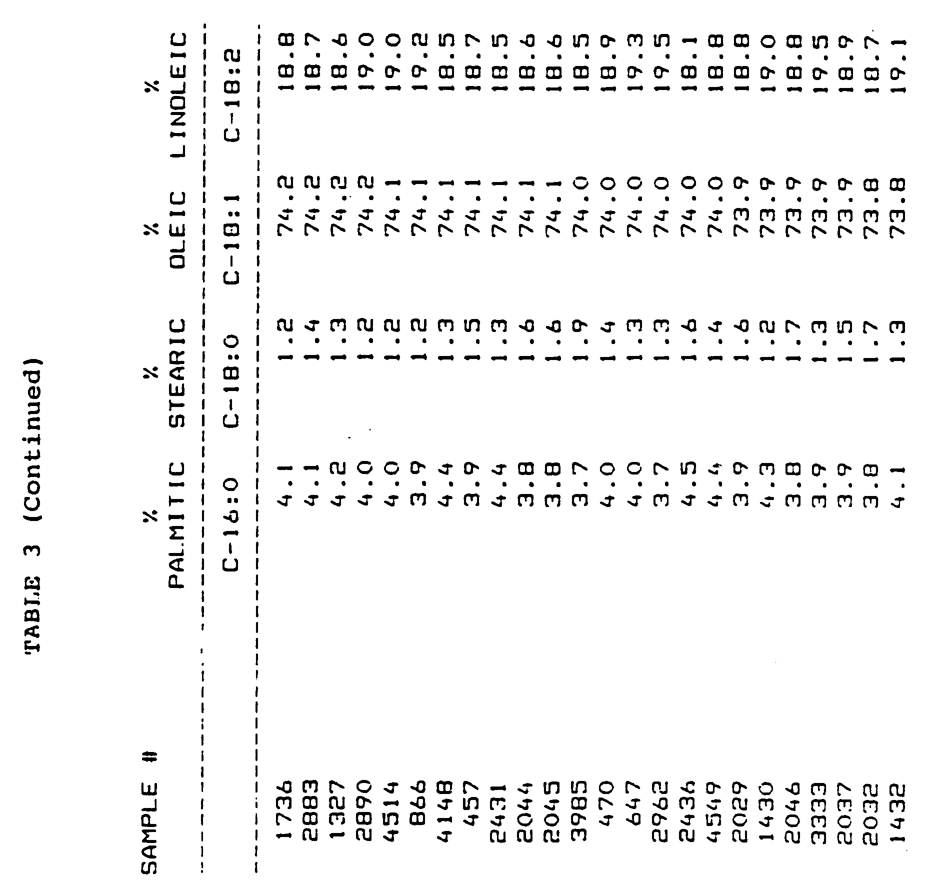

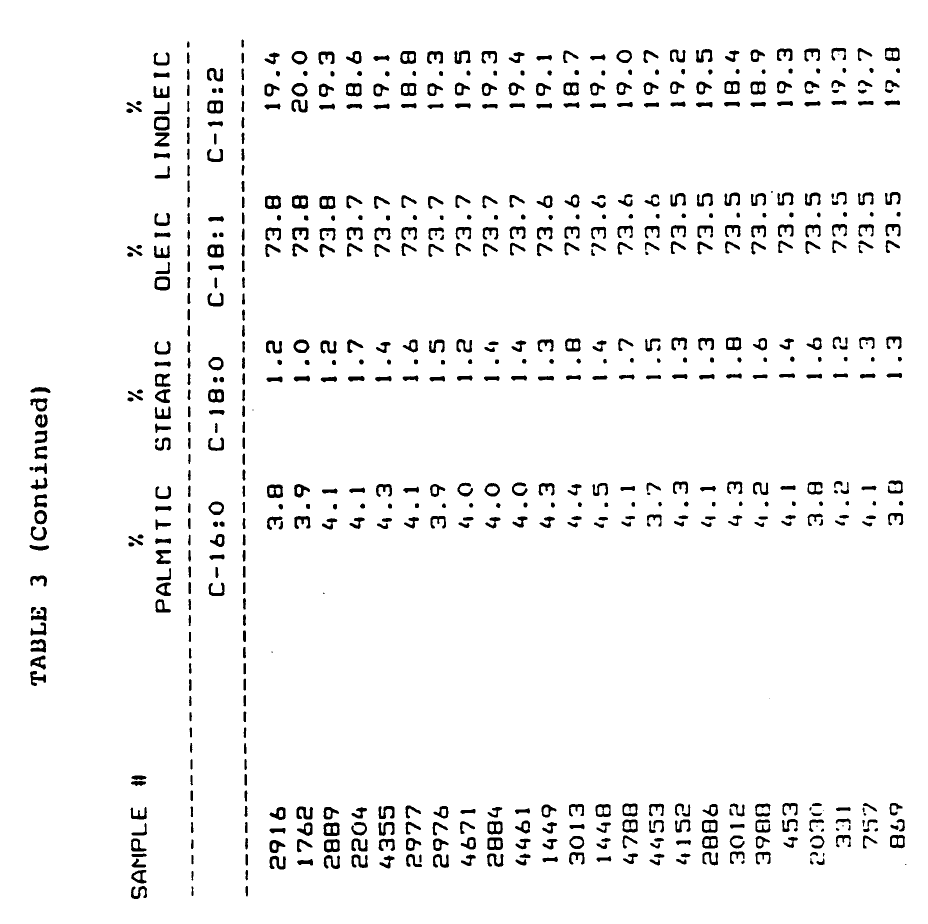

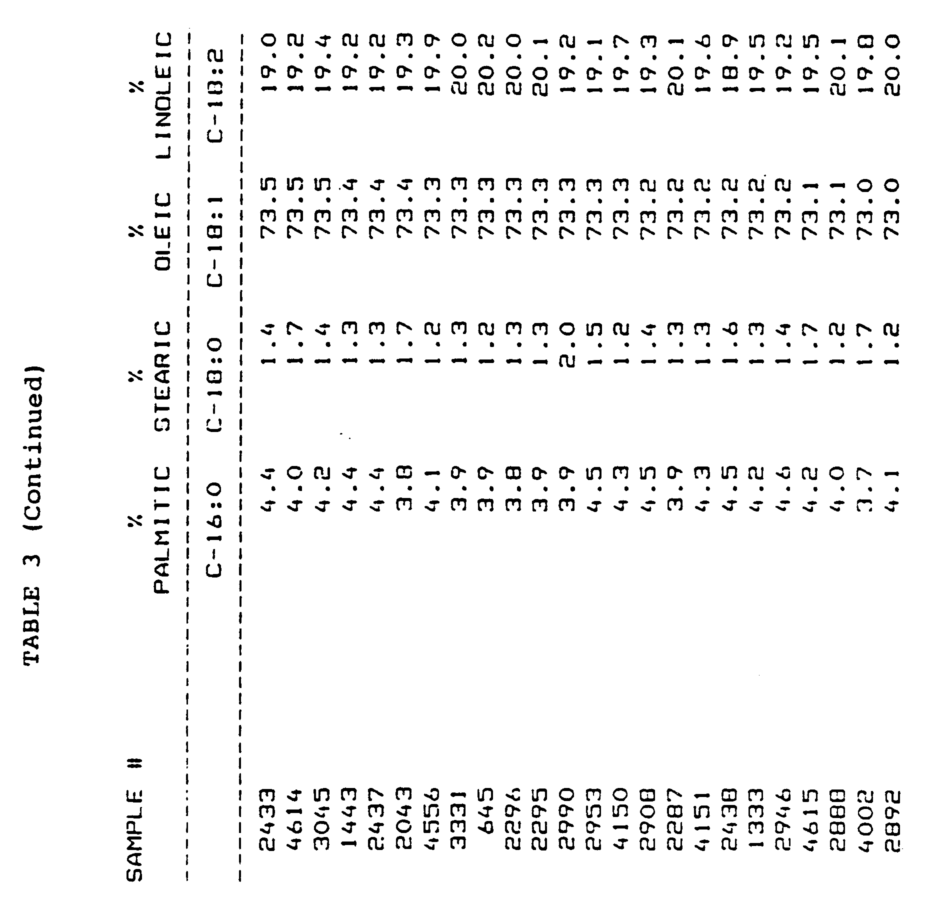

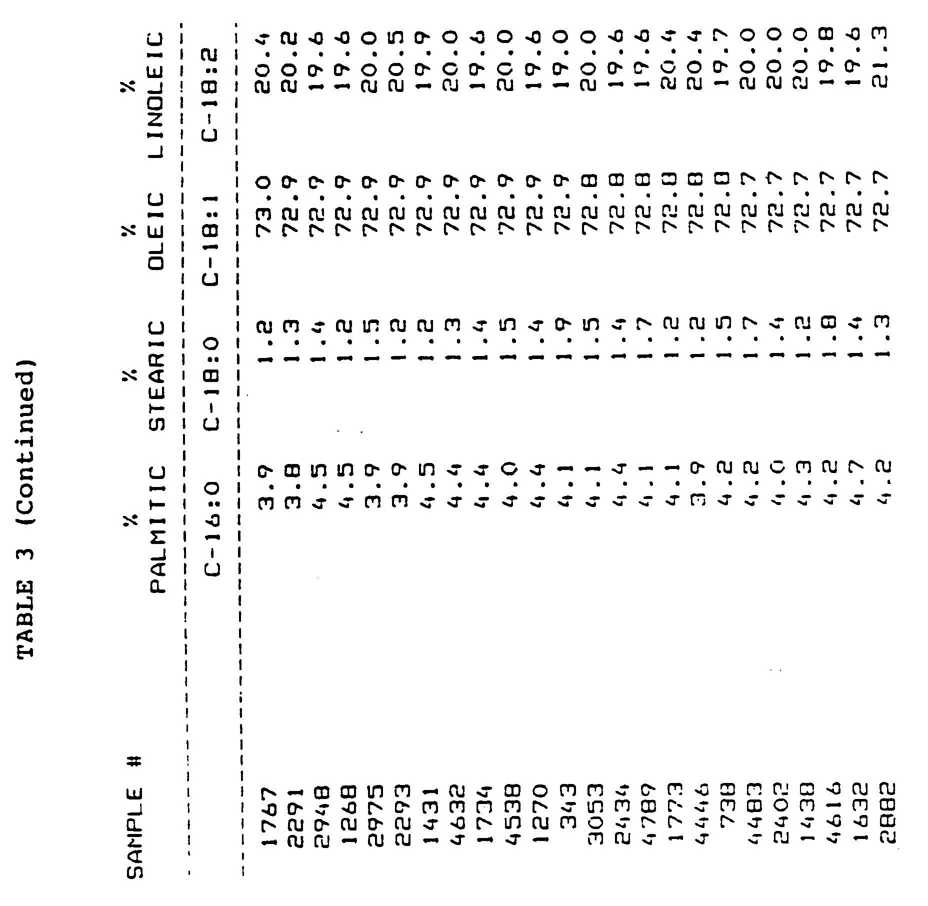

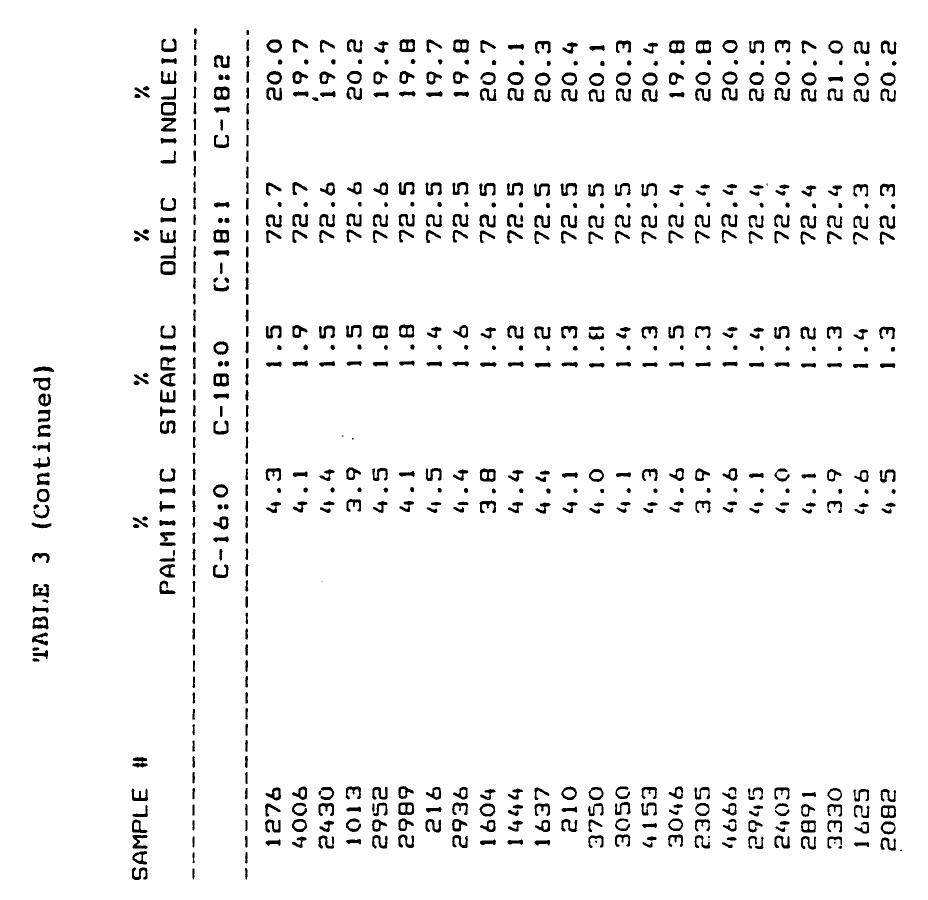

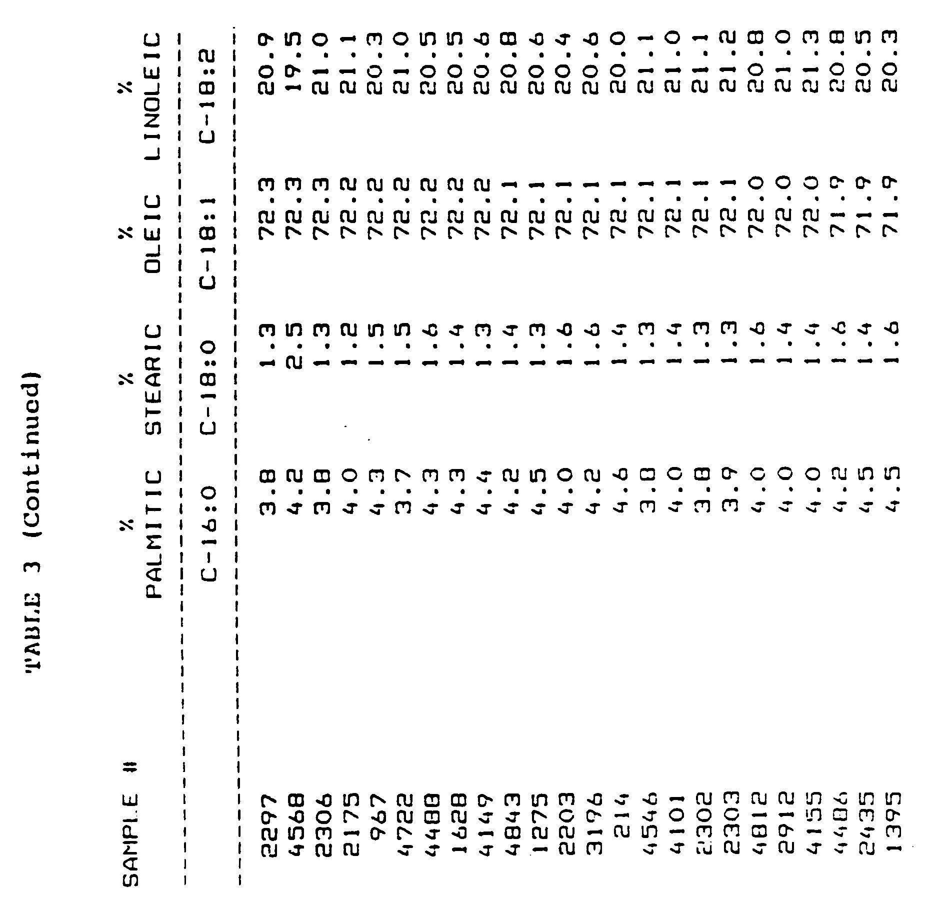

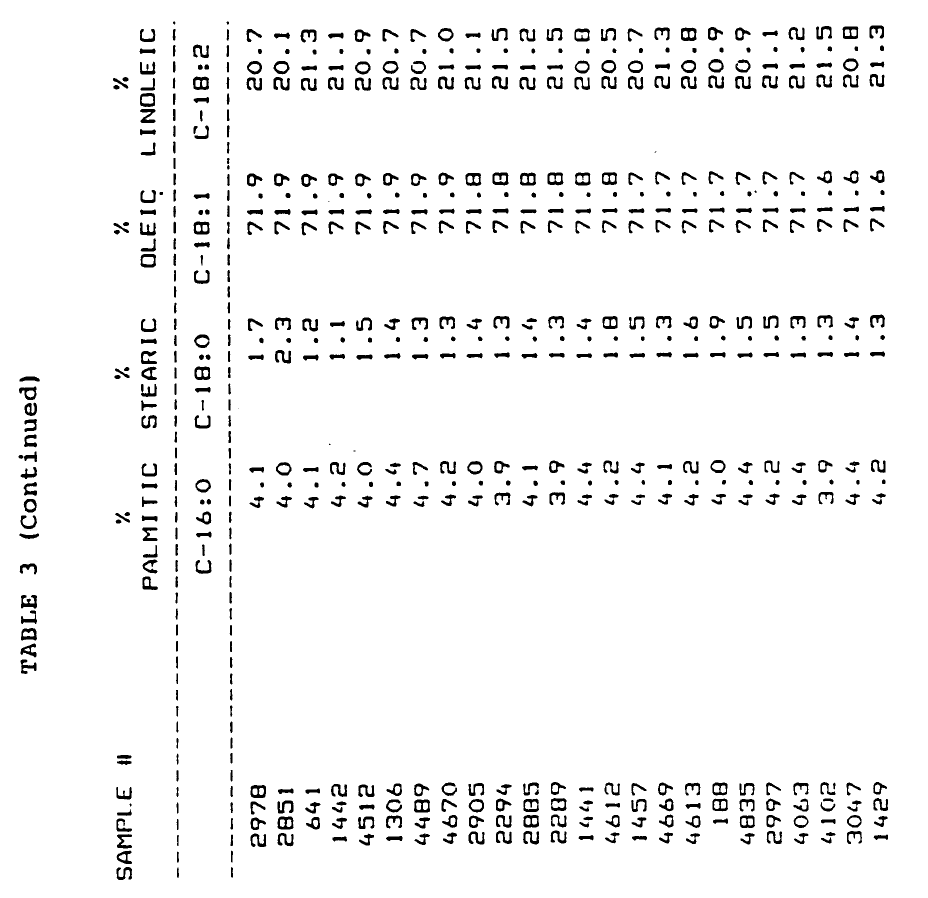

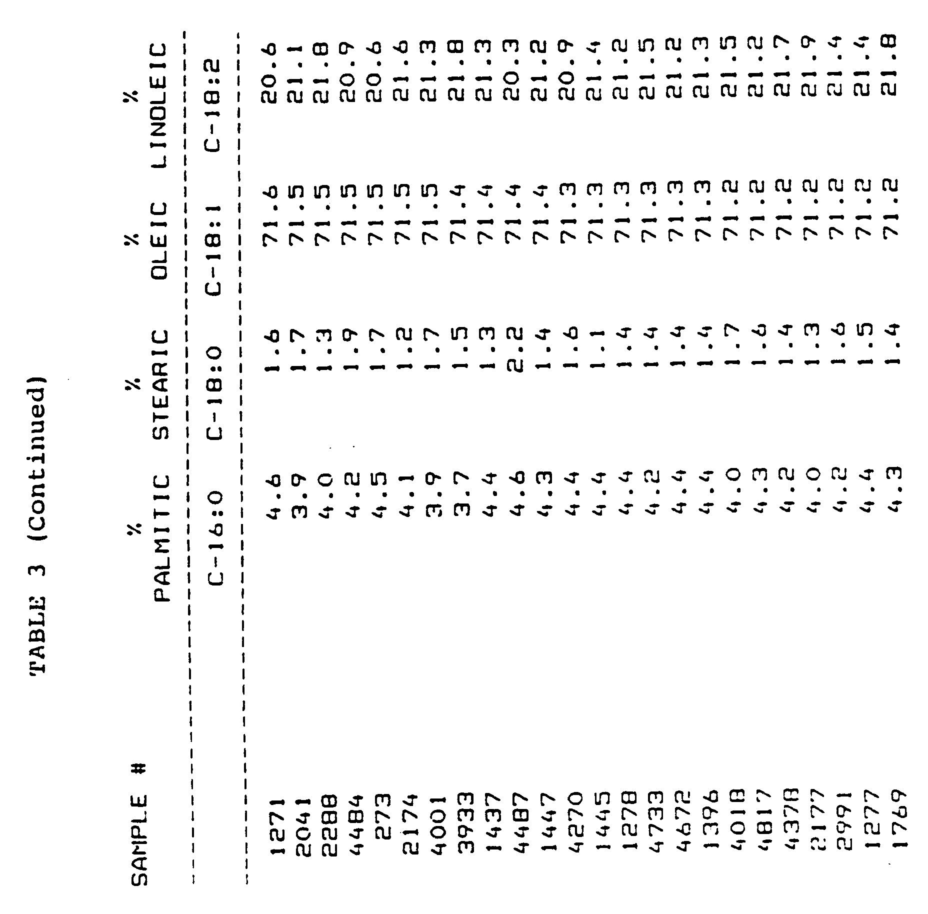

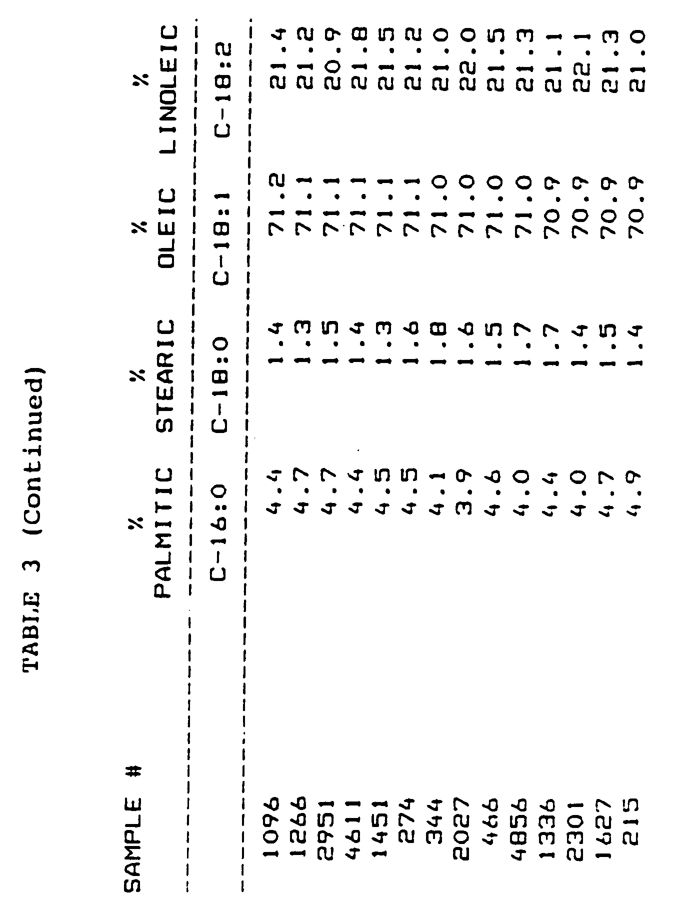

### Example 4

### 1986 Breeding Program and Results

These 302 lines were selfed in 1986 and the seed analyzed for oleic acid production. A total of 215 lines were selected after the 1986 season which produced seeds having an oleic acid content greater than 80%. The range in oleic acid content of these lines was 80% to 84.7%. The linoleic acid content ranged from 8.4% to 13.6% and the saturated fatty acid content ranged from 3.9% to 7.5%. The ratio of saturated fatty acids to oleic acid ranged from 0.047 to 0.086, with one line at 0.093. Twenty-four of these lines produced seeds having an oleic acid content of greater than 83%. The mean temperatures for July and August were 68.9°F (20.5°C) and 68.0°F (20.0°C) respectively. The results are shown in Table 4.

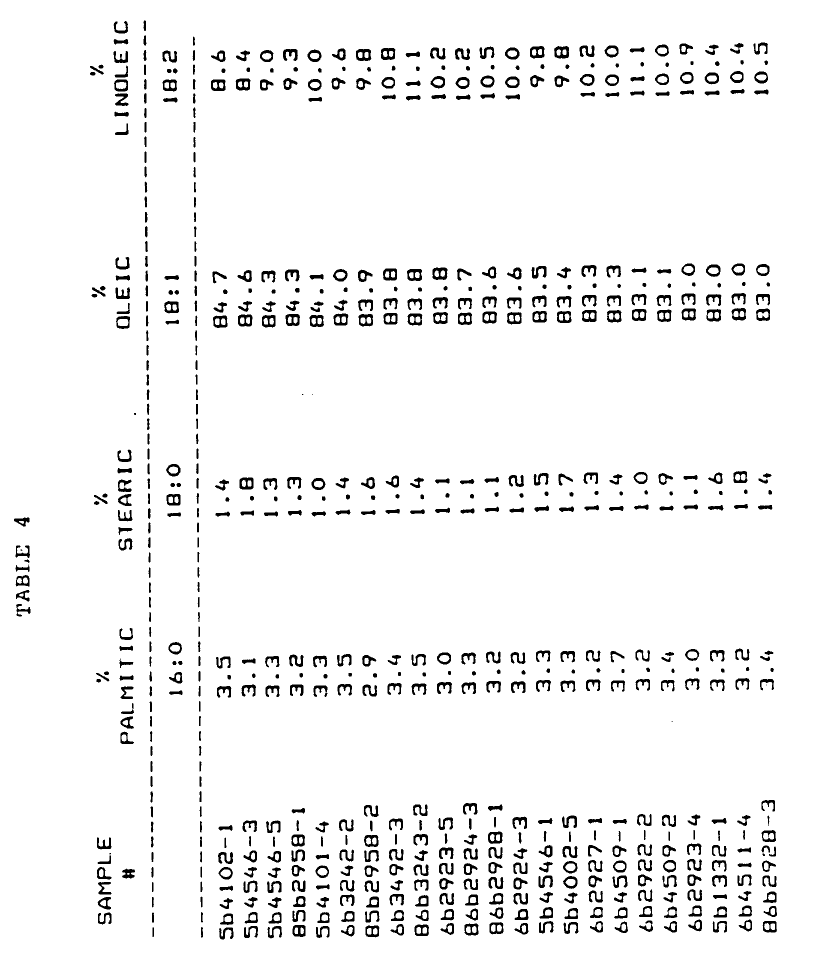

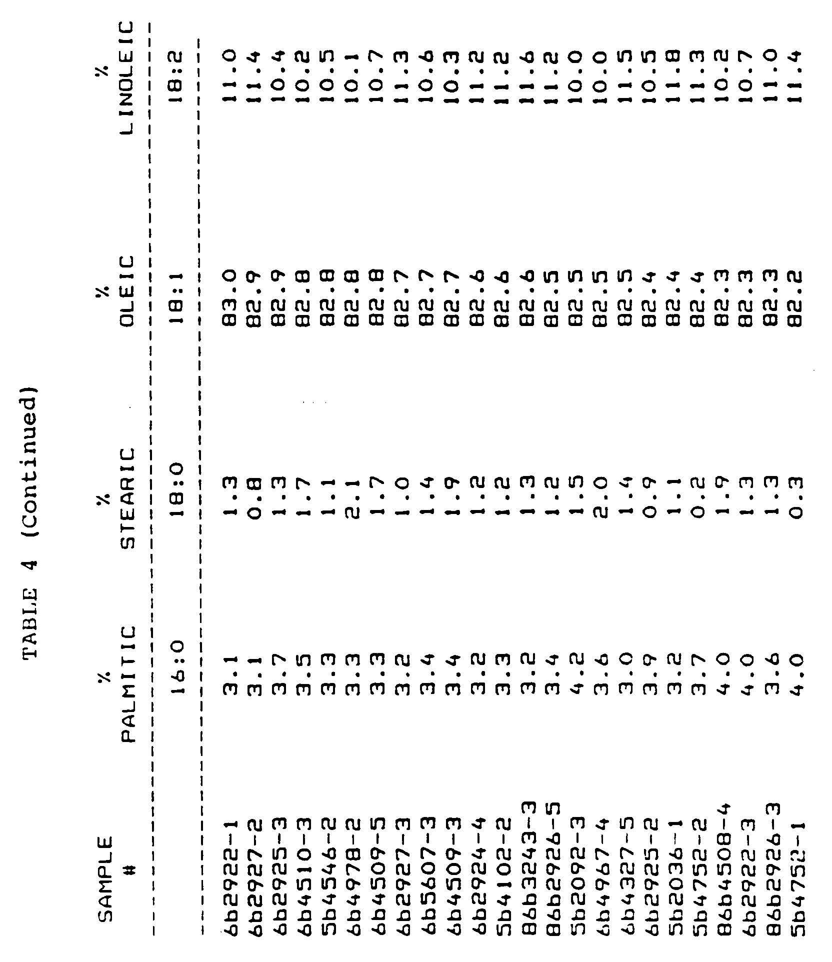

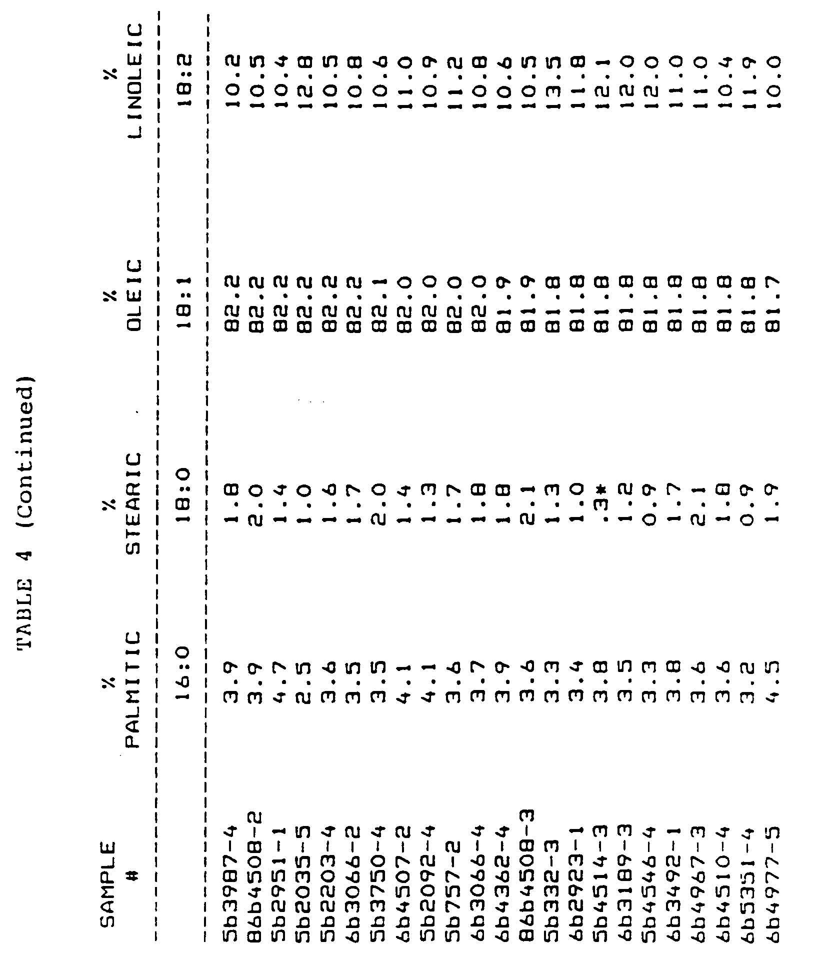

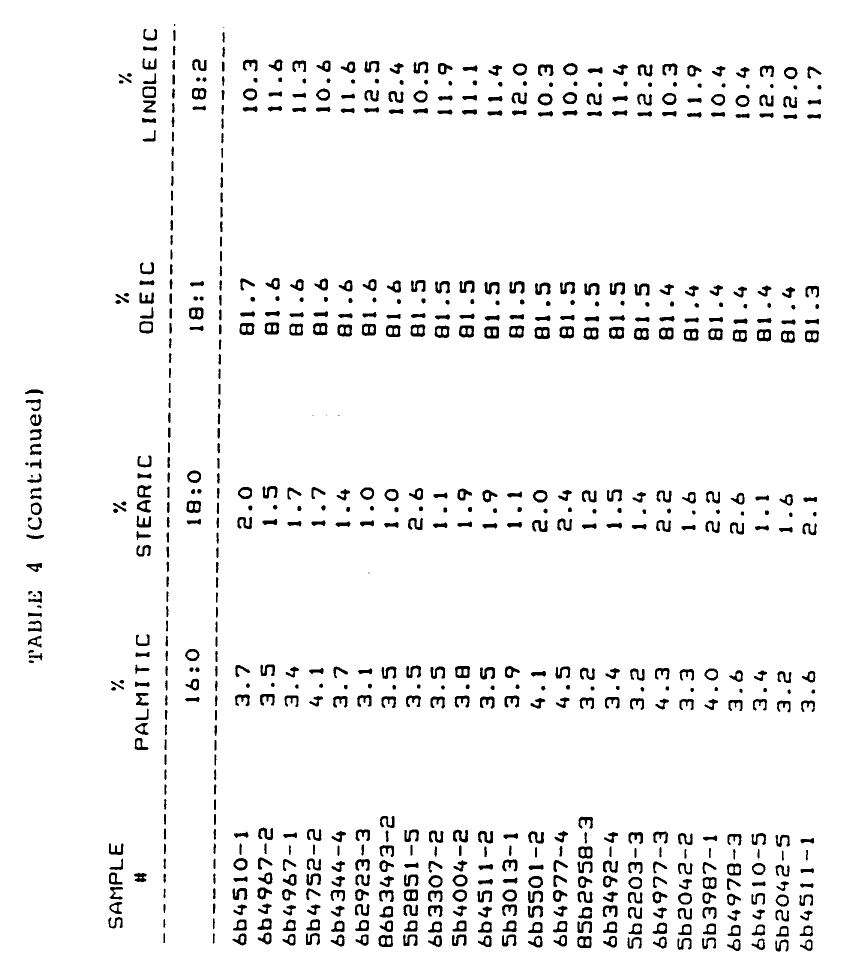

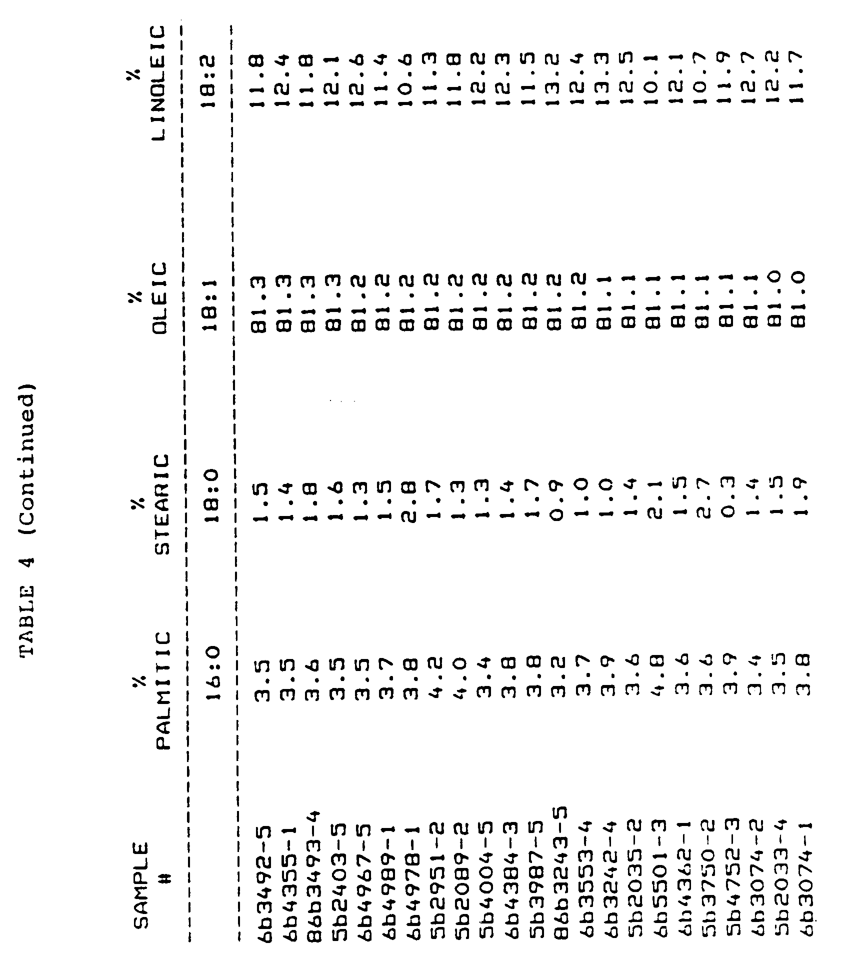

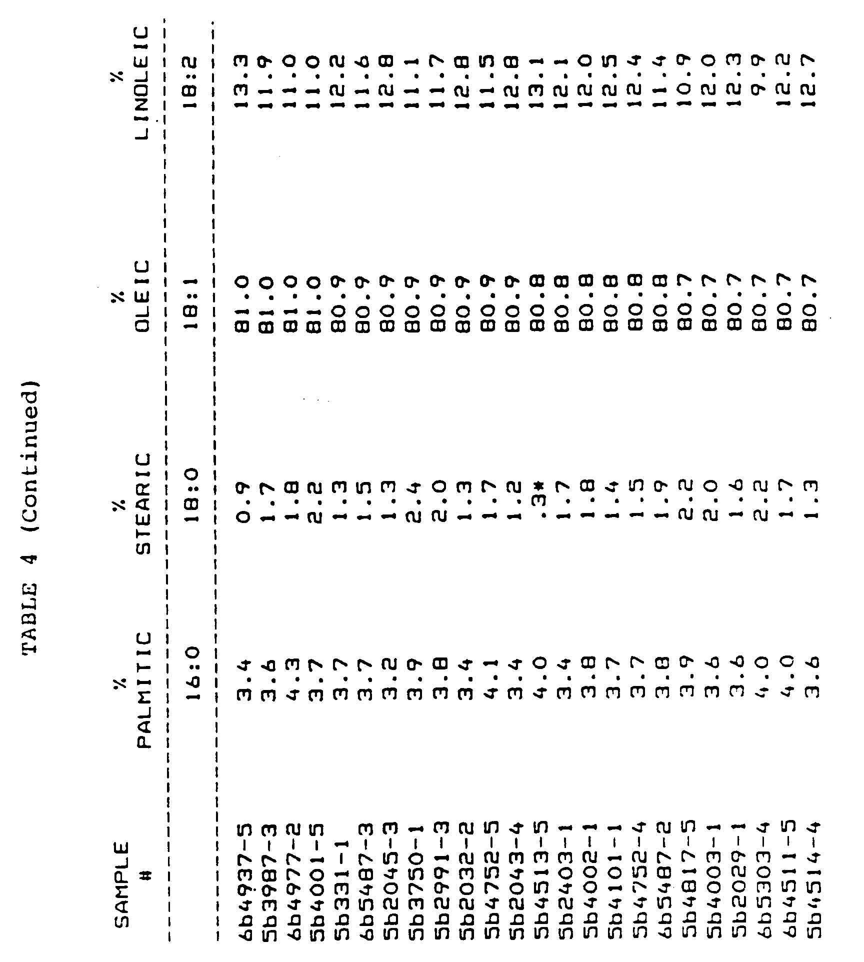

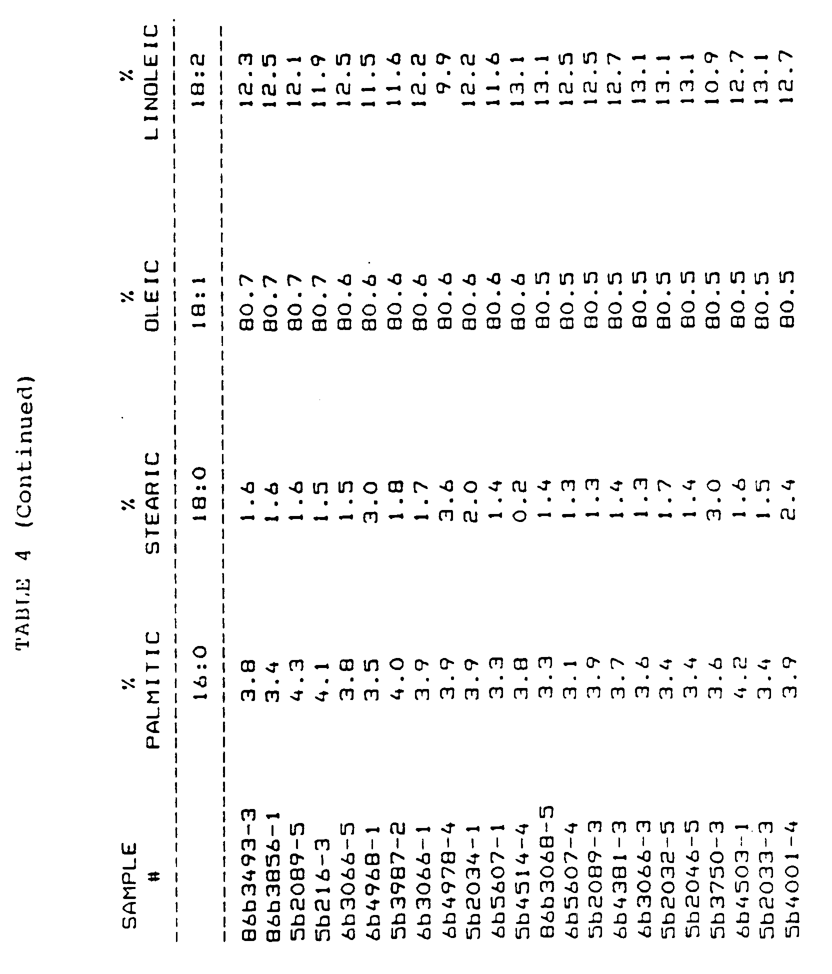

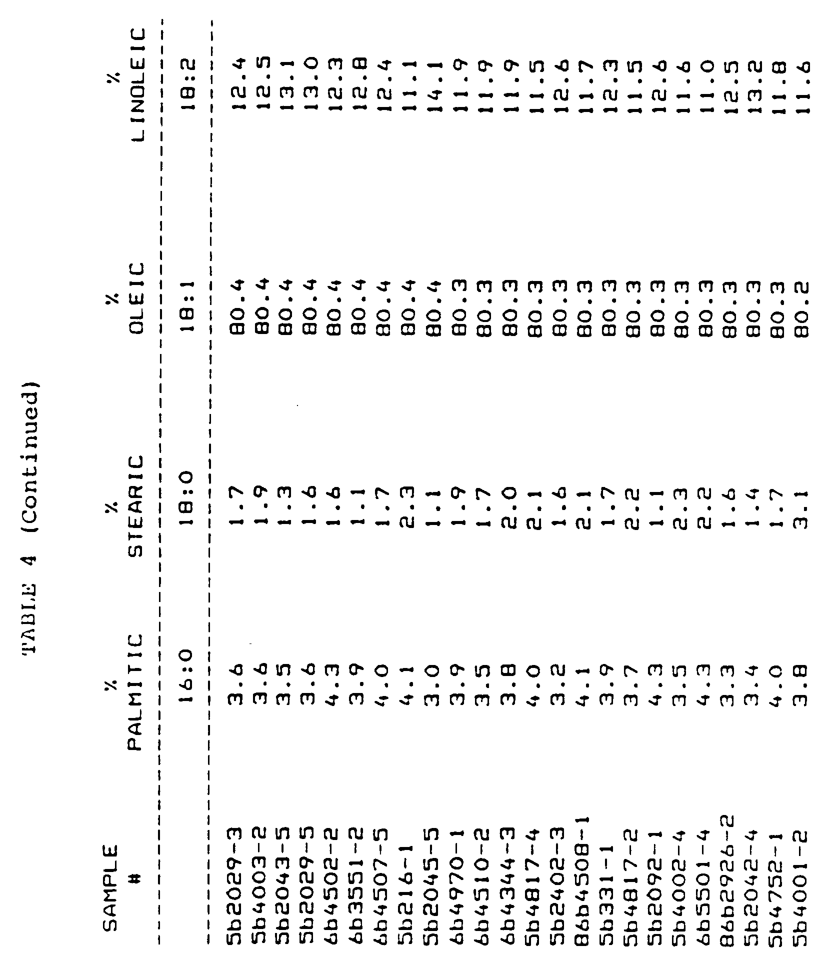

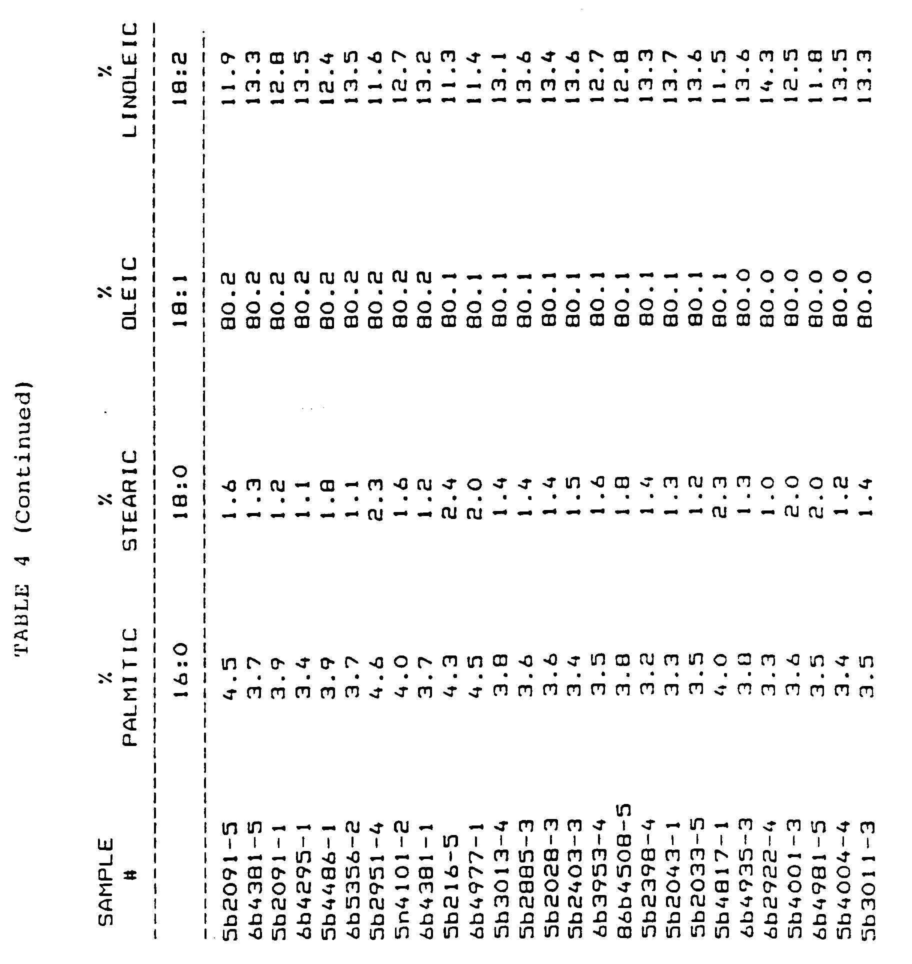

### Example 5

### 1987 Breeding Program and Results

These 215 lines were selfed in 1987 and the seed analyzed for oleic acid production. A total of 303 lines were selected after the 1987 season which produced seeds having oleic acid content greater than 80%. The range in oleic acid content of these lines was 81.9% to 85.9%. The linoleic acid content ranged from 7.2% to 11.8% and the saturated fatty acid content ranged from 3.7% to 6.2%. The ratio of saturated fatty acids to oleic acid ranged from 0.044 to 0.076. Forty-eight of these lines produced seeds having an oleic acid content of greater than 84%. The mean temperatures for July and August were 71.3°F (21.8°C) and 66.0°F (18.9°C), respectively. The results are shown in Table 5.

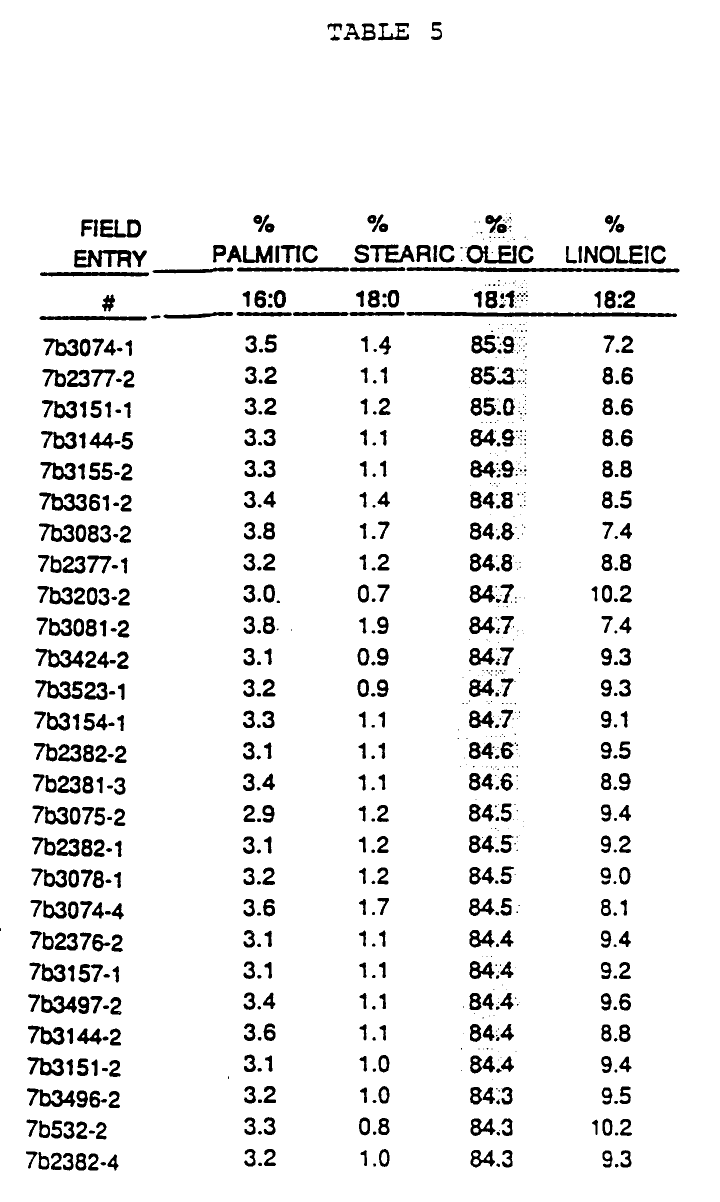

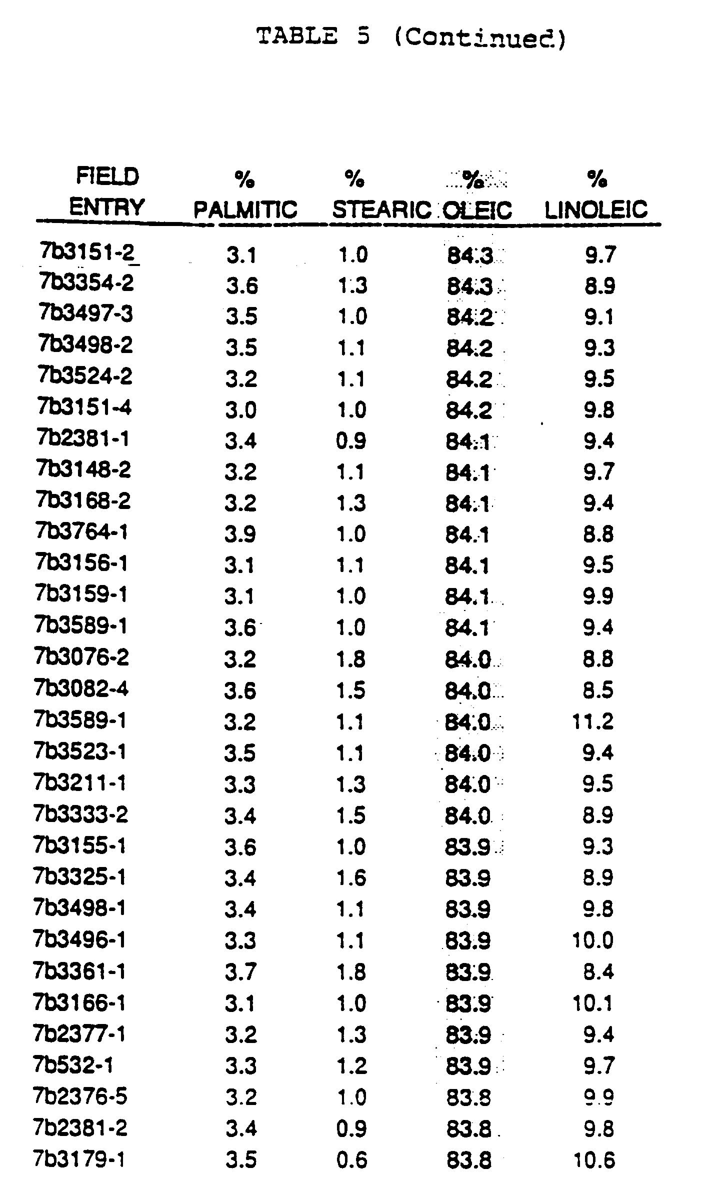

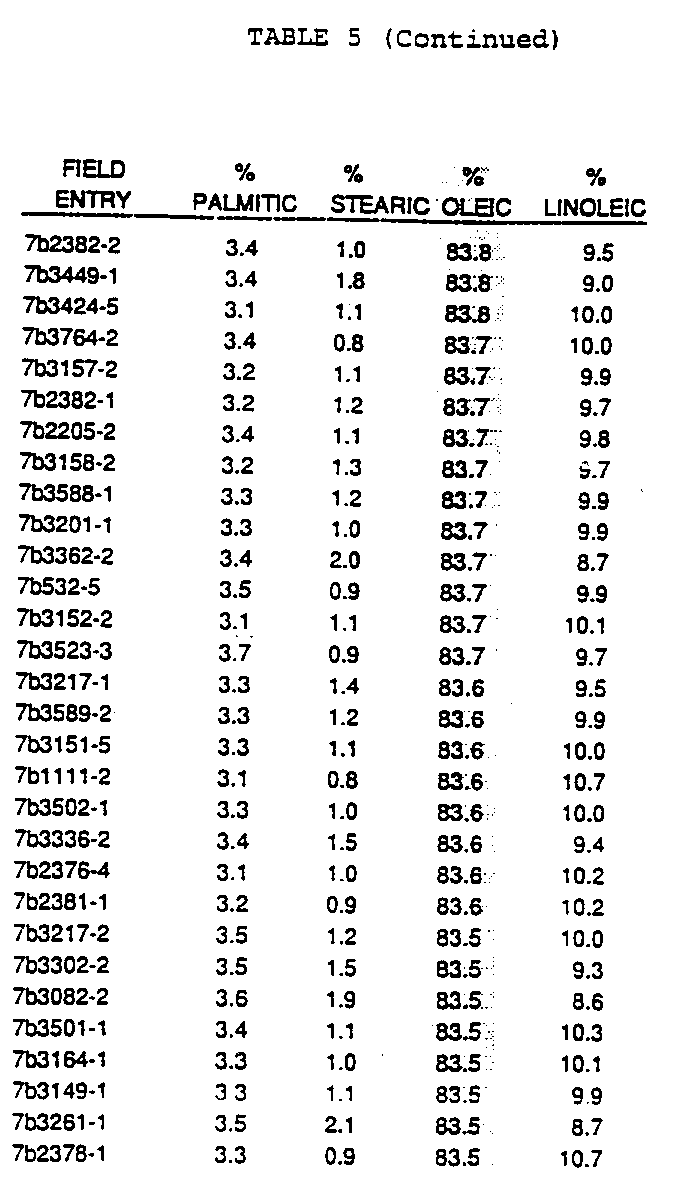

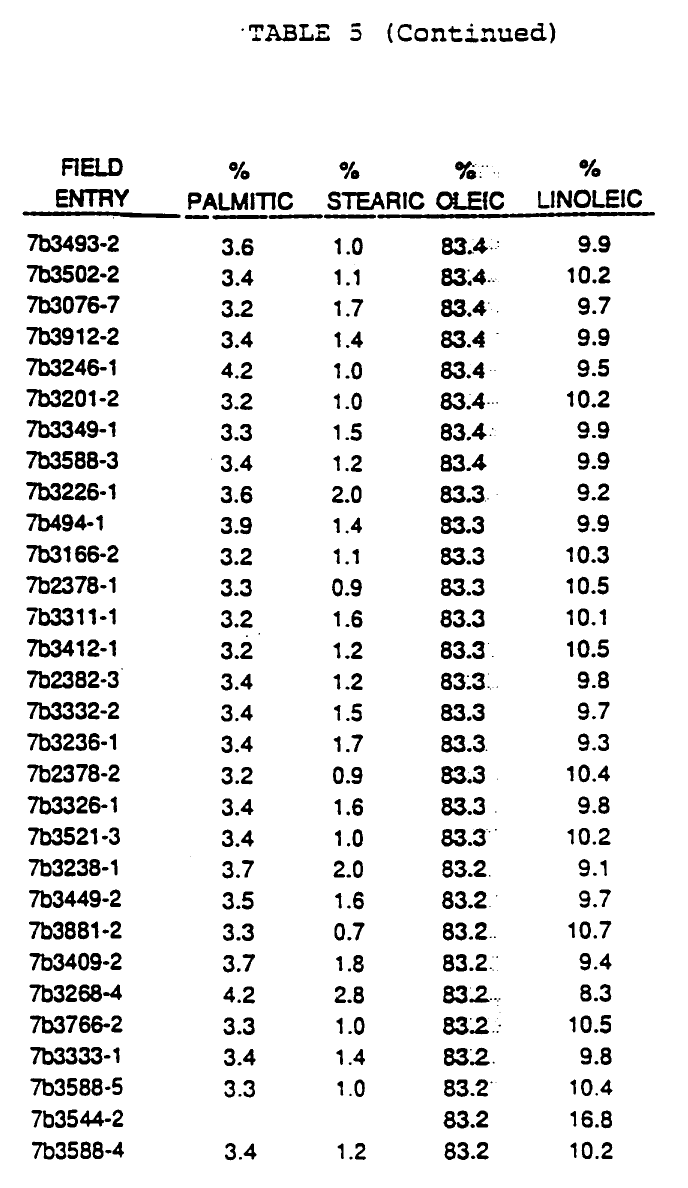

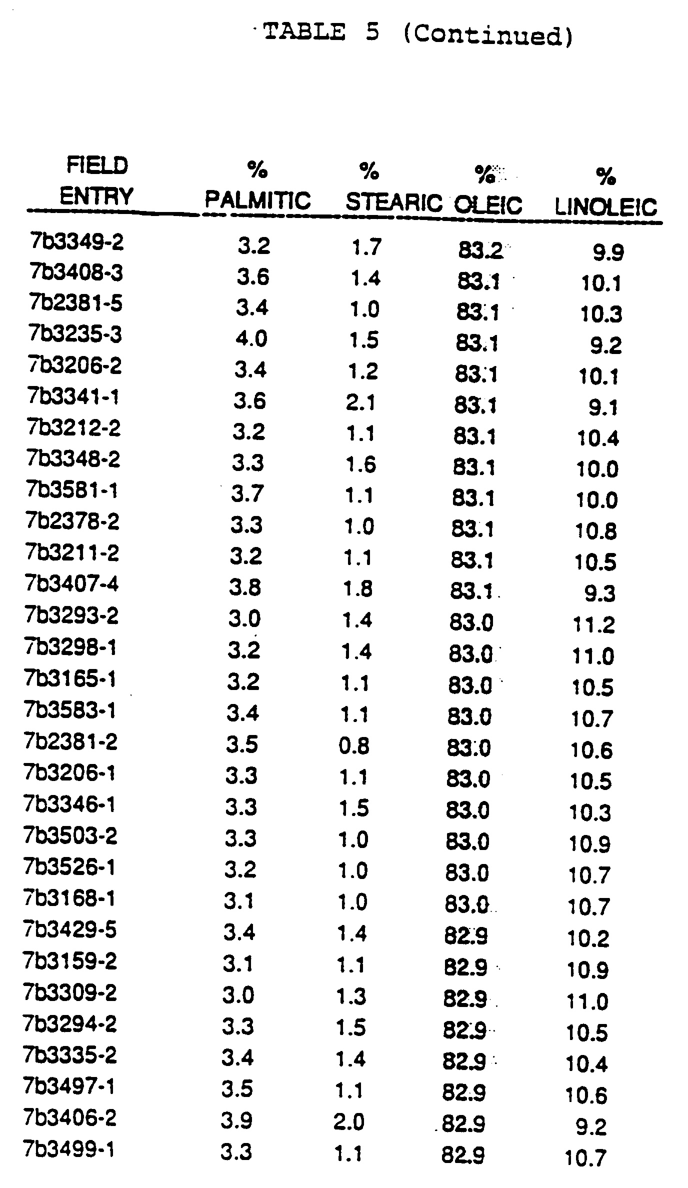

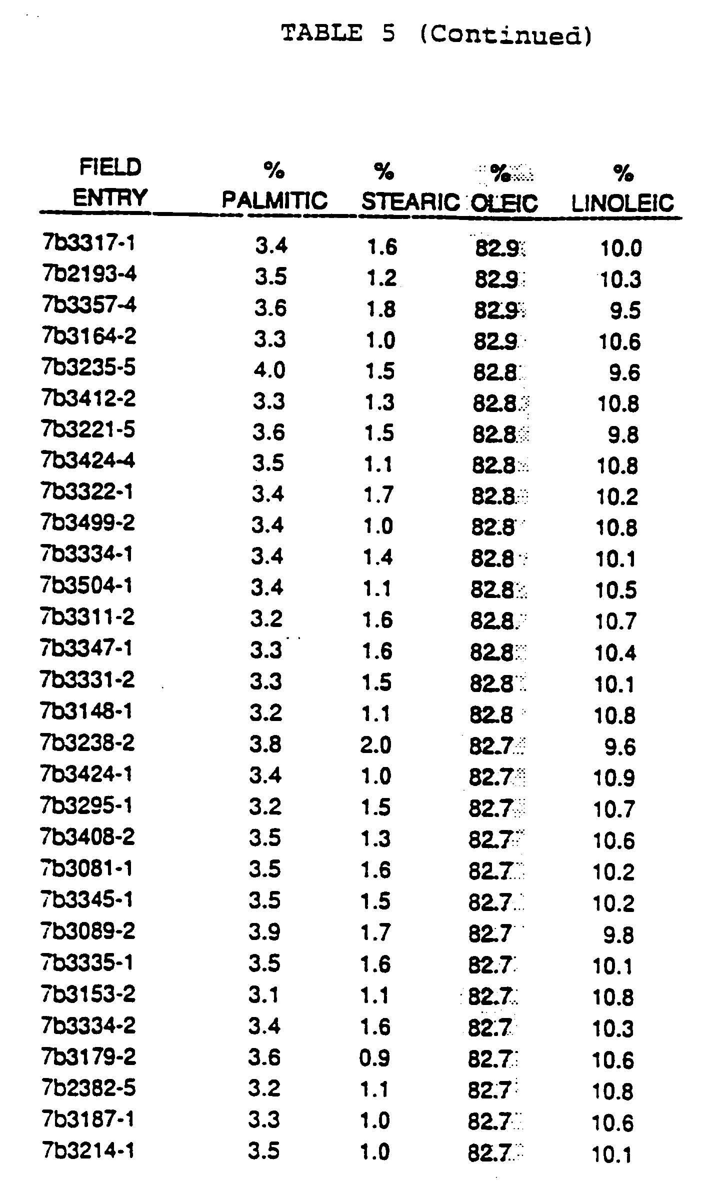

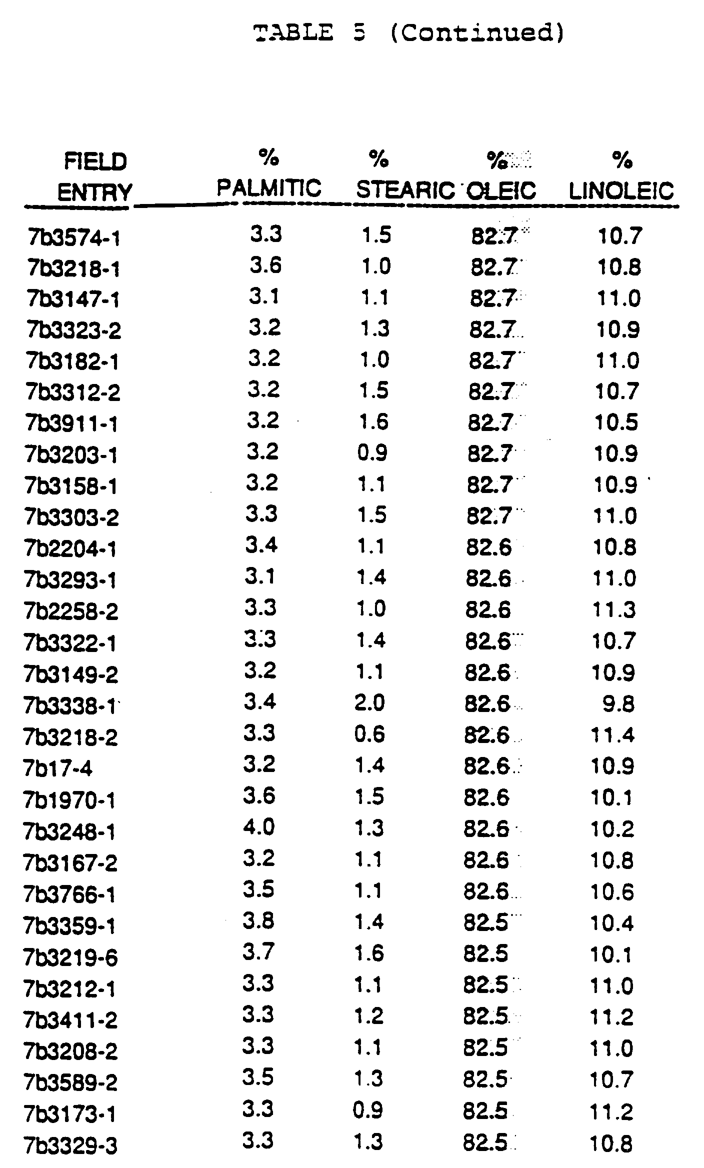

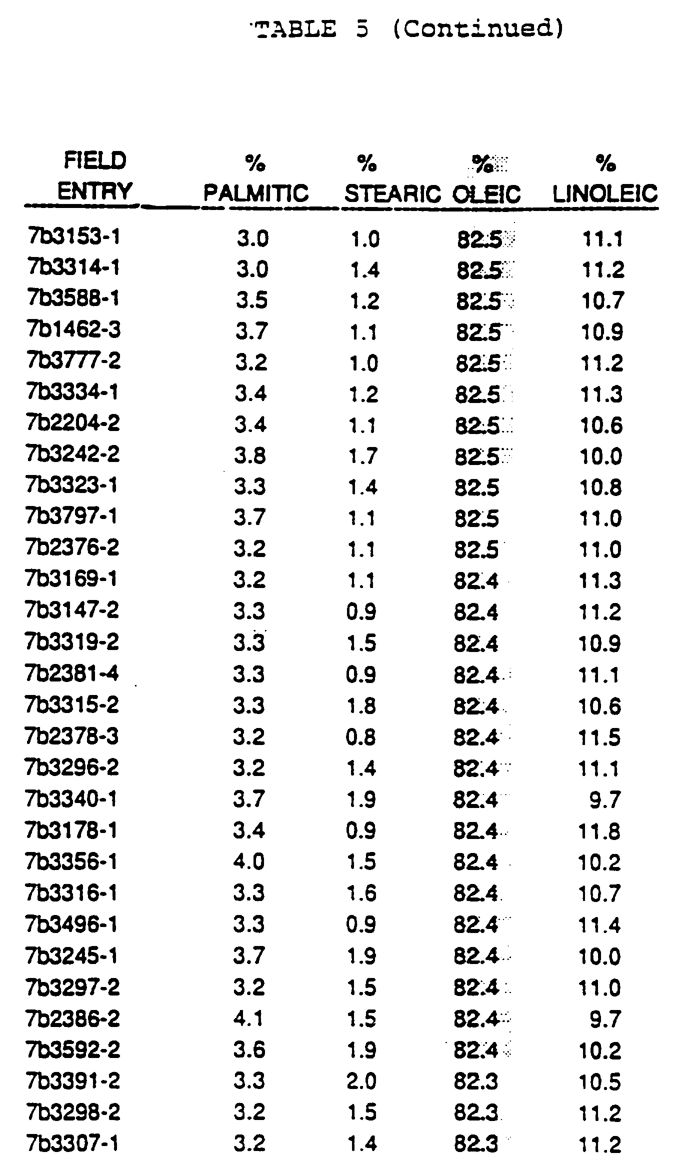

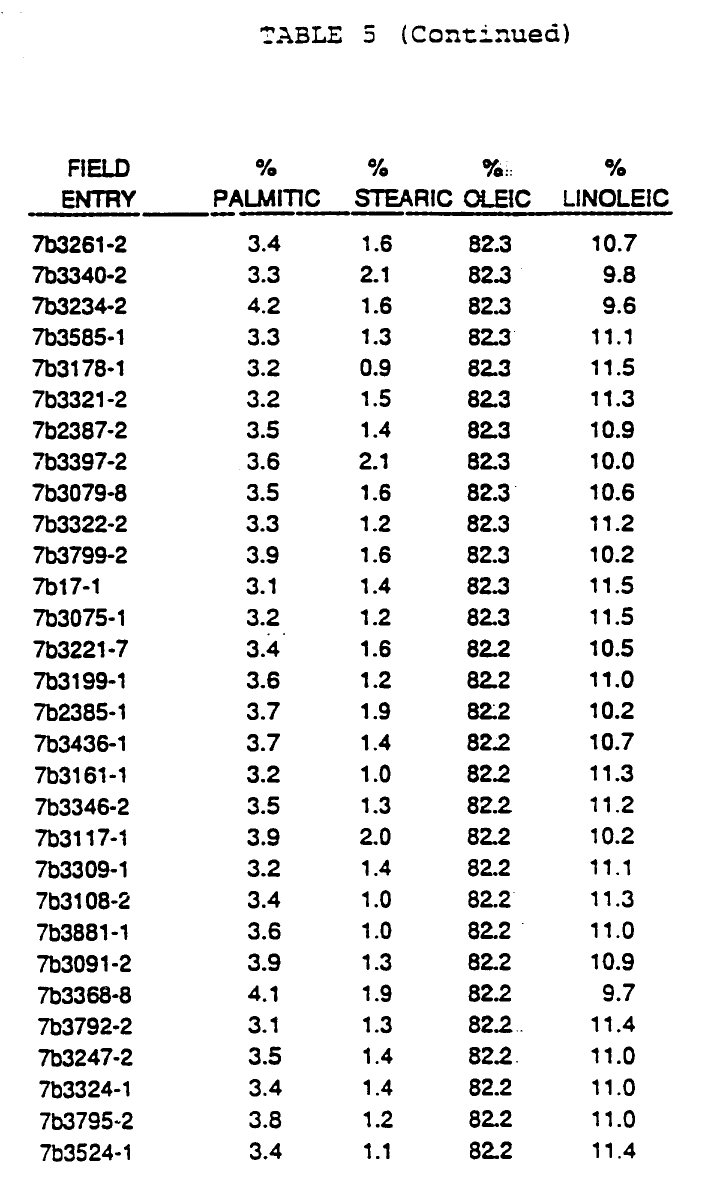

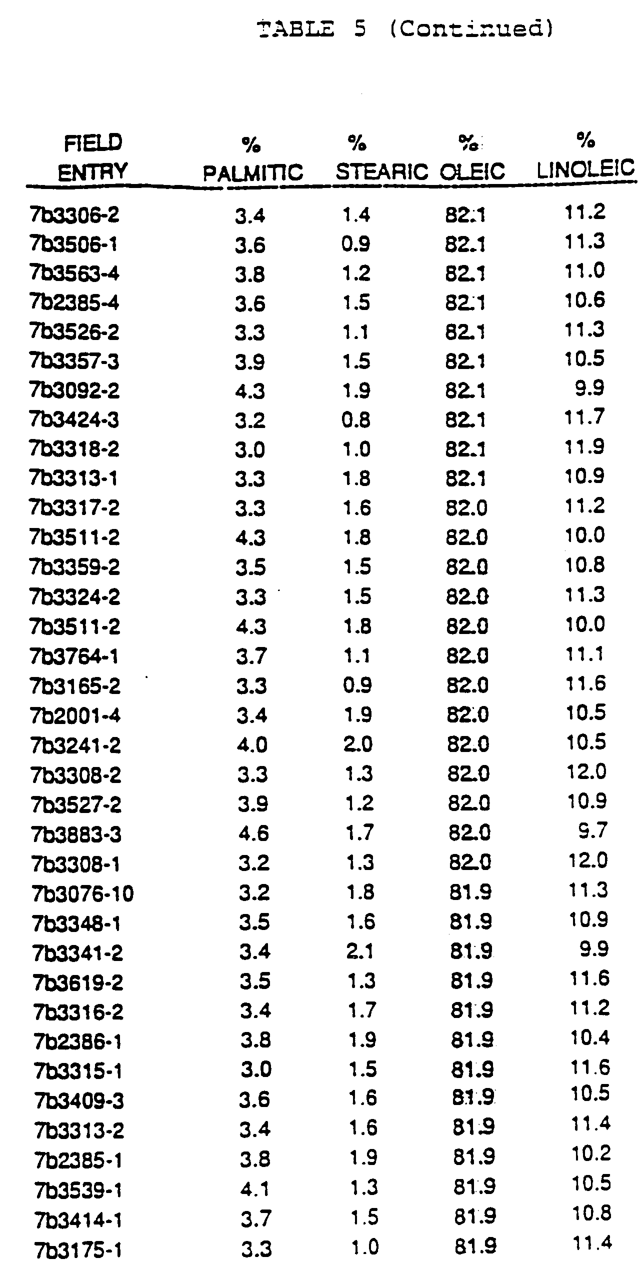

### Example 6

### 1988 Breeding Program and Results

These 303 lines were selfed in 1988 and the seed analyzed for oleic acid production. A total of 352 lines were selected after the 1988 season which produced seeds having oleic acid content greater than 80%. The range in oleic acid content of these lines was 83.4% to 86.1%. The linoleic acid content ranged from 7.4% to 10.4% and the saturated fatty acid content ranged from 4.7% to 5.8%, with six lines up to 6.5%. The ratio of saturated fatty acids to oleic acid ranged from 0.055 to 0.070, with three lines up to 0.075. Twenty-two of these lines produced seeds having an oleic acid content of greater than 85%. The mean temperatures for July and August were 73.7°F (23.2°C) and about 70.6°F (21.4°C), respectively. The results are shown in Table 6.

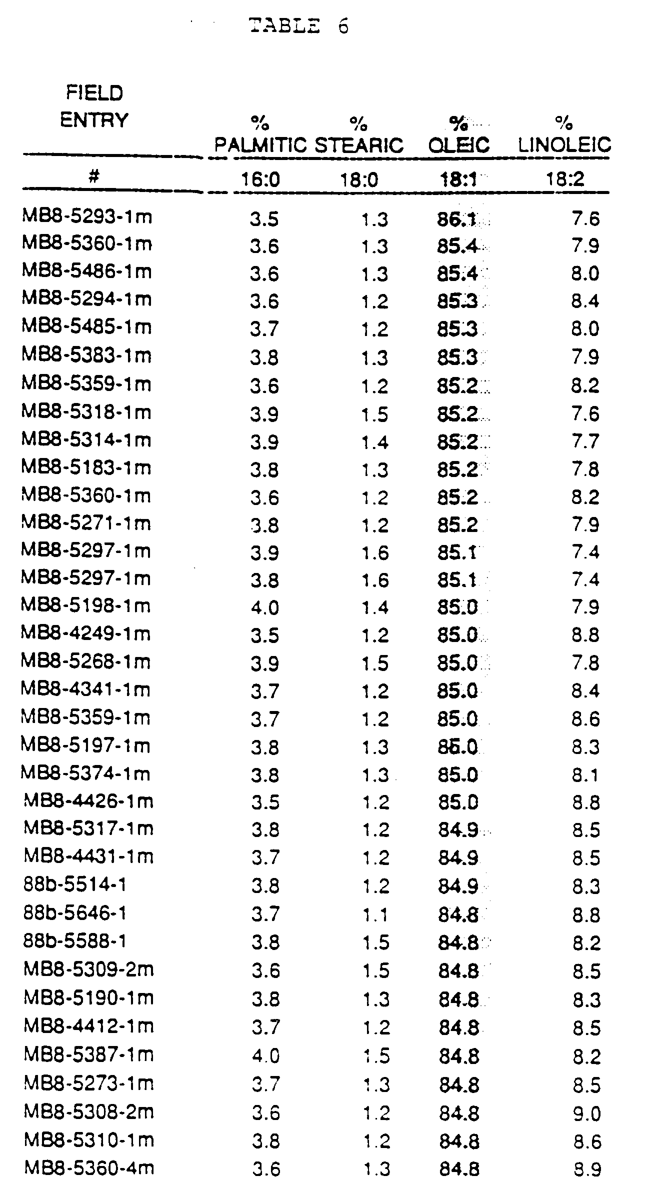

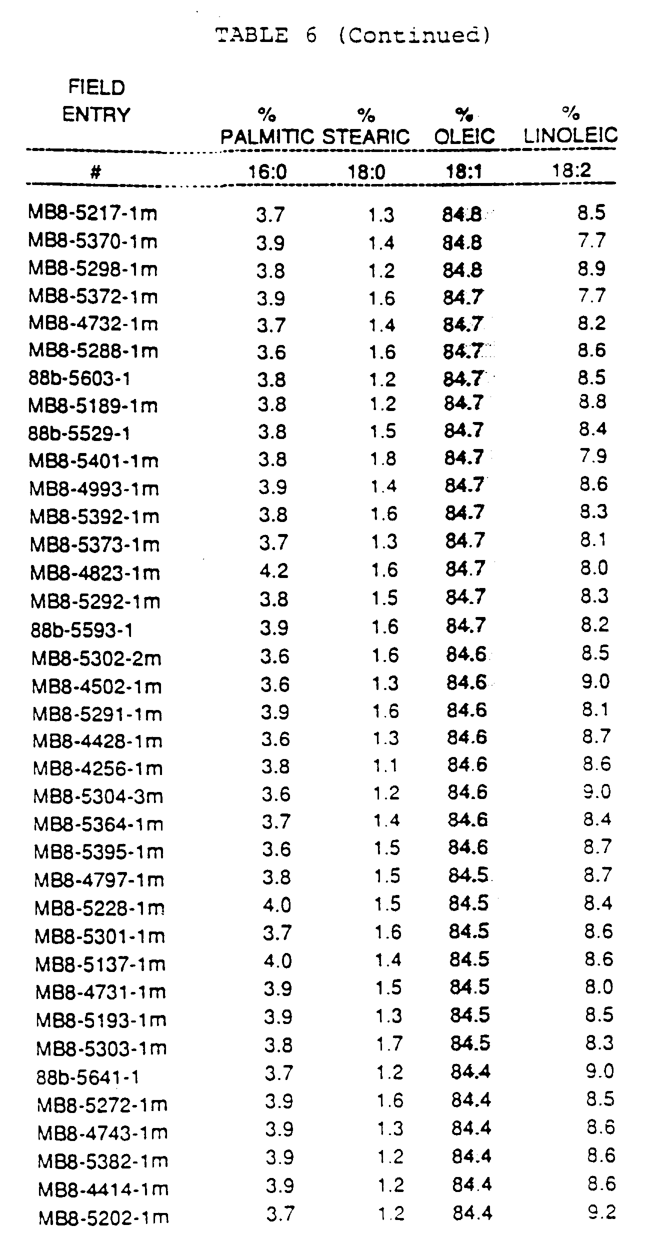

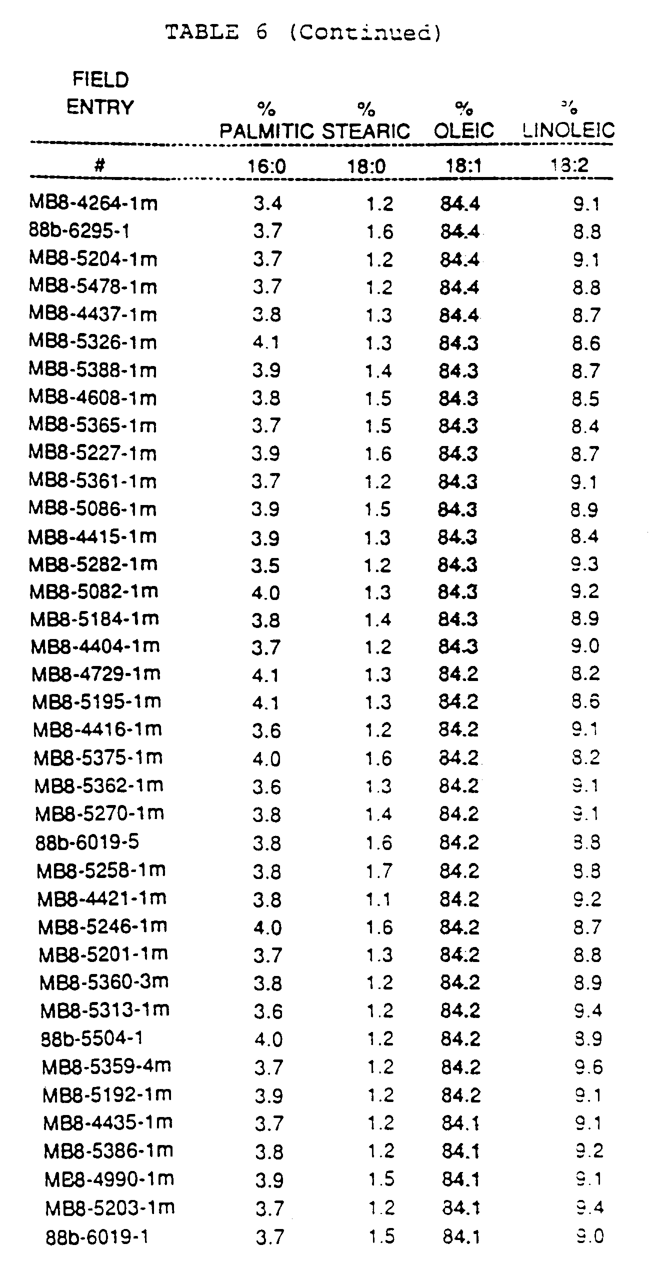

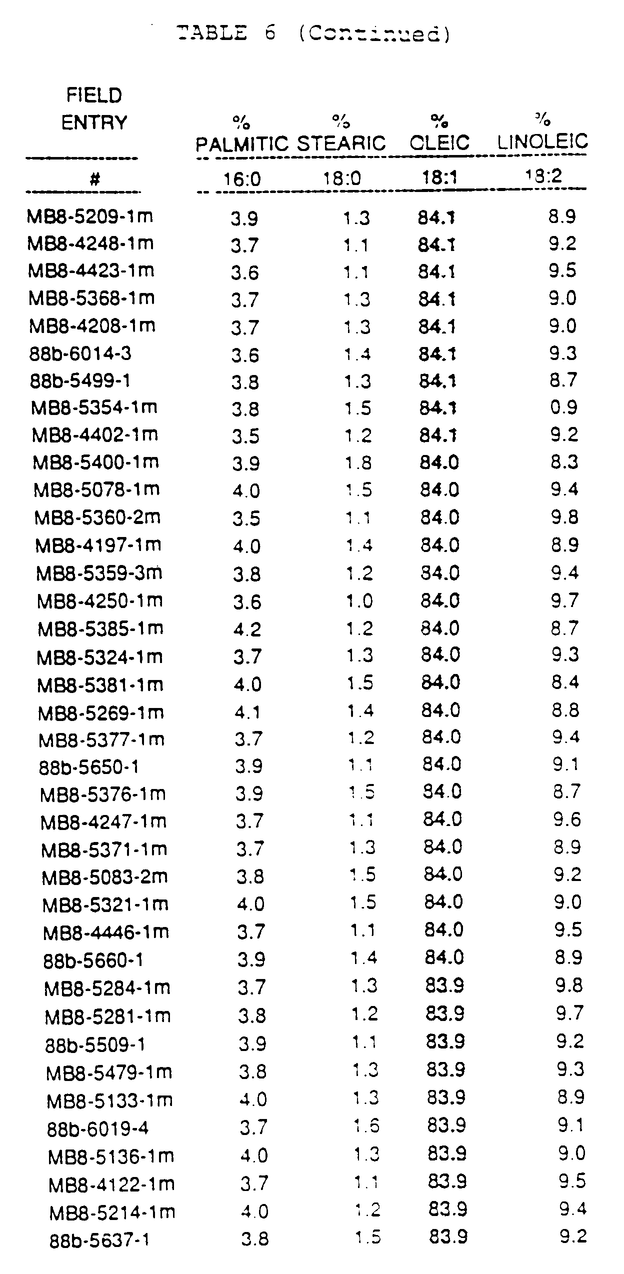

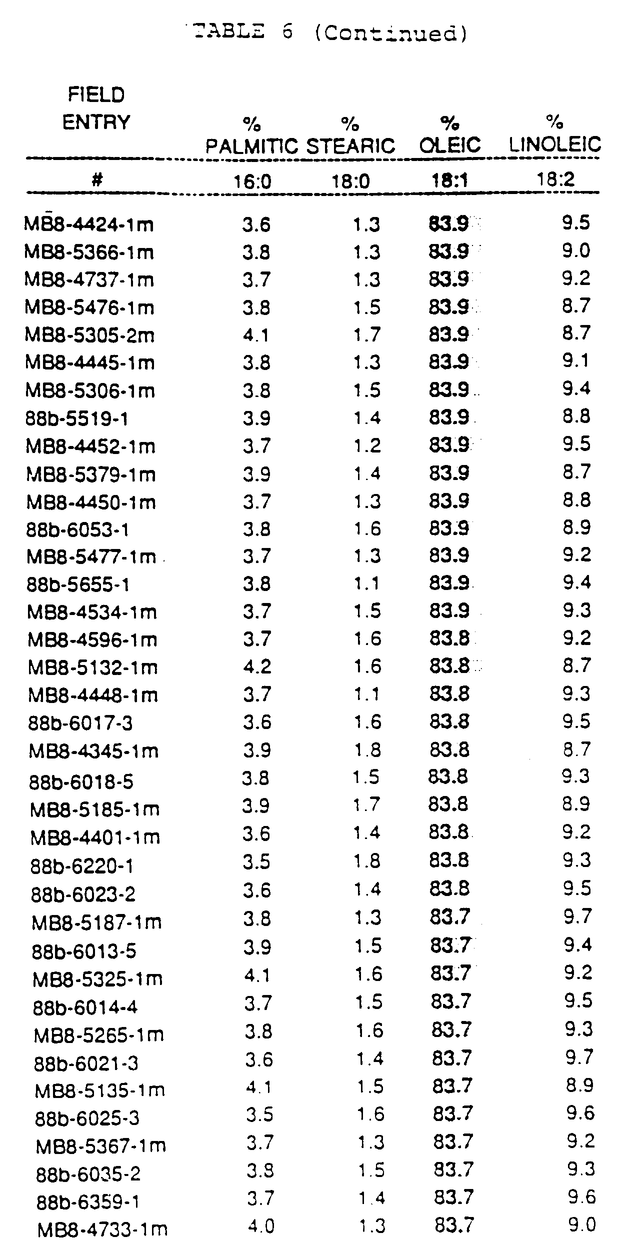

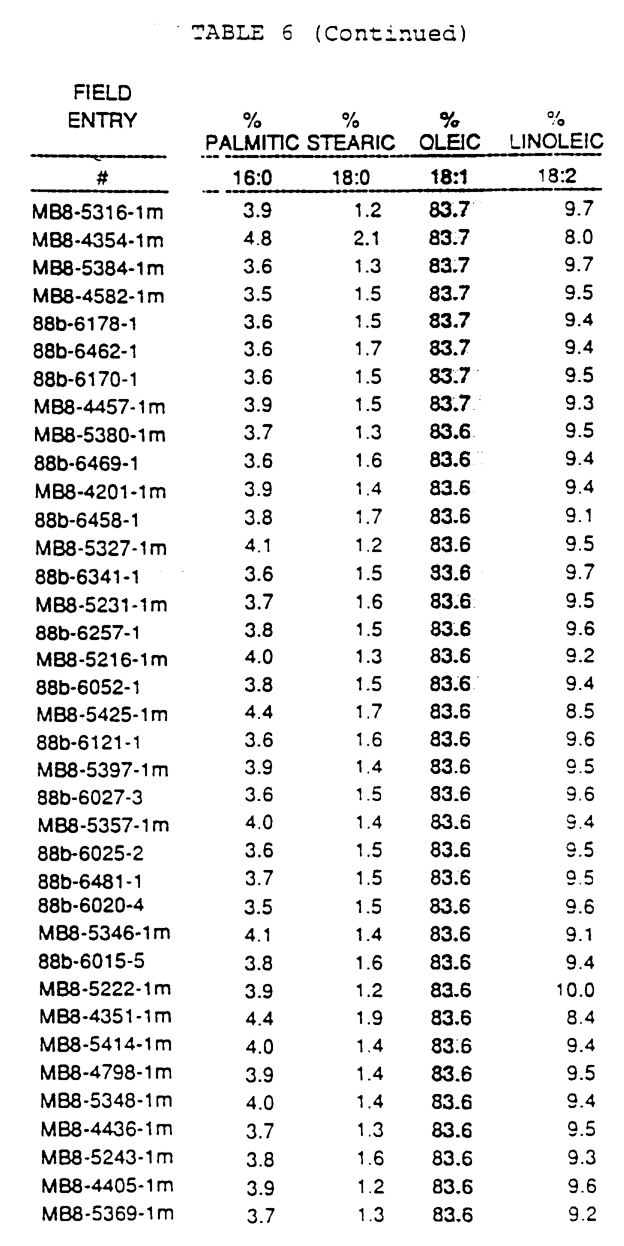

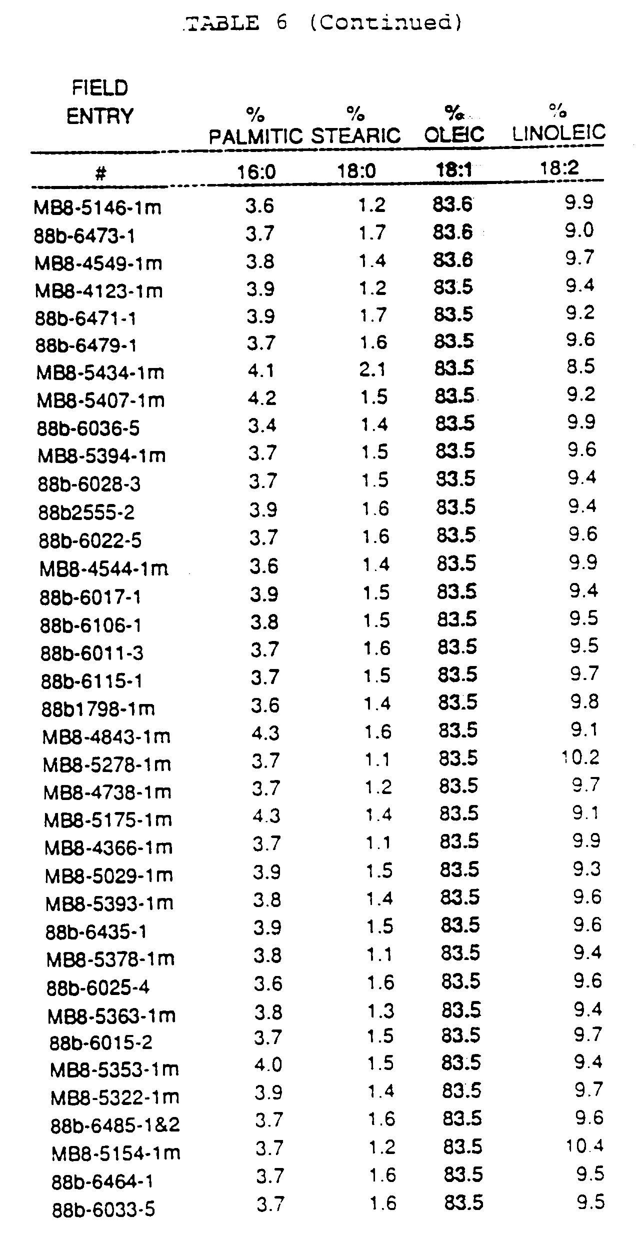

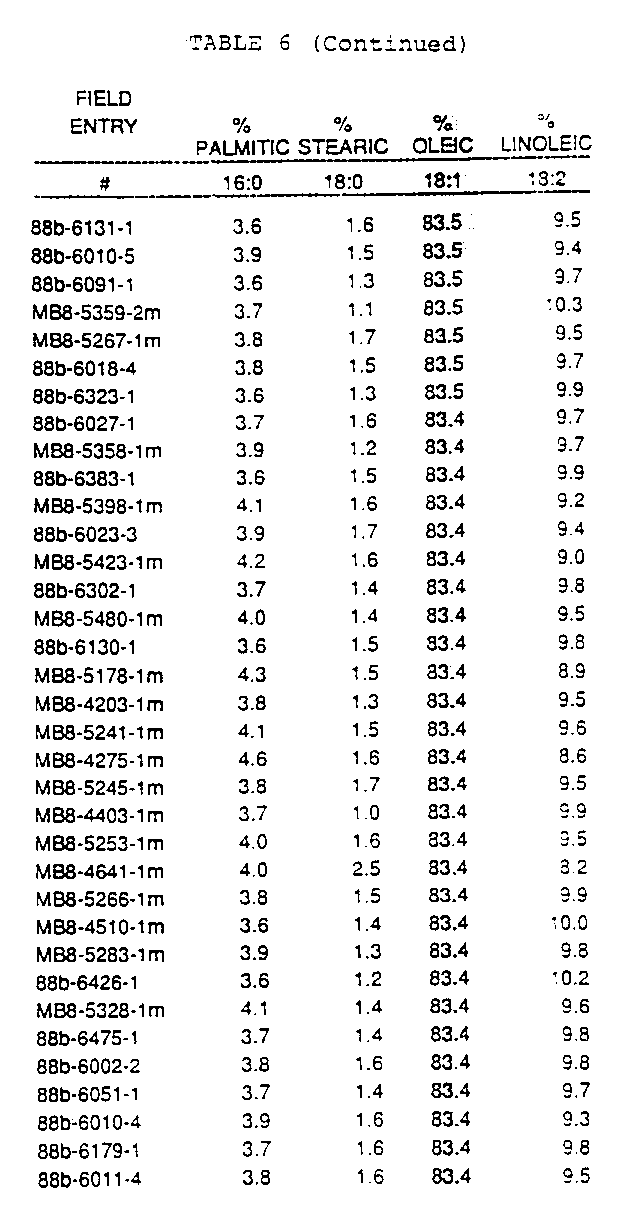

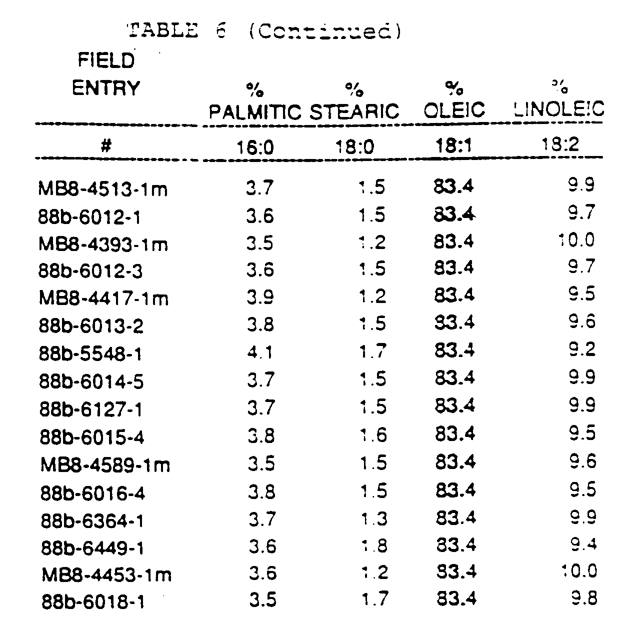

These 352 lines were selfed in 1989 and the seed analyzed for oleic acid production. Slight changes seen in the oleic acid content of the progeny with some lines approaching 87%. The mean temperature for July and August was 75.6°F (24.2°C) and about 70.8°F (21.6°C) respectively. In order to increase the oleic acid content to greater than 87% and up to 90%, the previously obtained lines are intercrossed to derive new lines. These new lines produce seed having an oleic acid content of up to 90%.

### Example 7

### Uniformity of Oil Production

Several lines were followed between 1985 and 1988 to demonstrate the stability and inheritability of the high oleic acid trait. These lines were grown and the seed harvested and analyzed each year. The results are shown in Table 7.

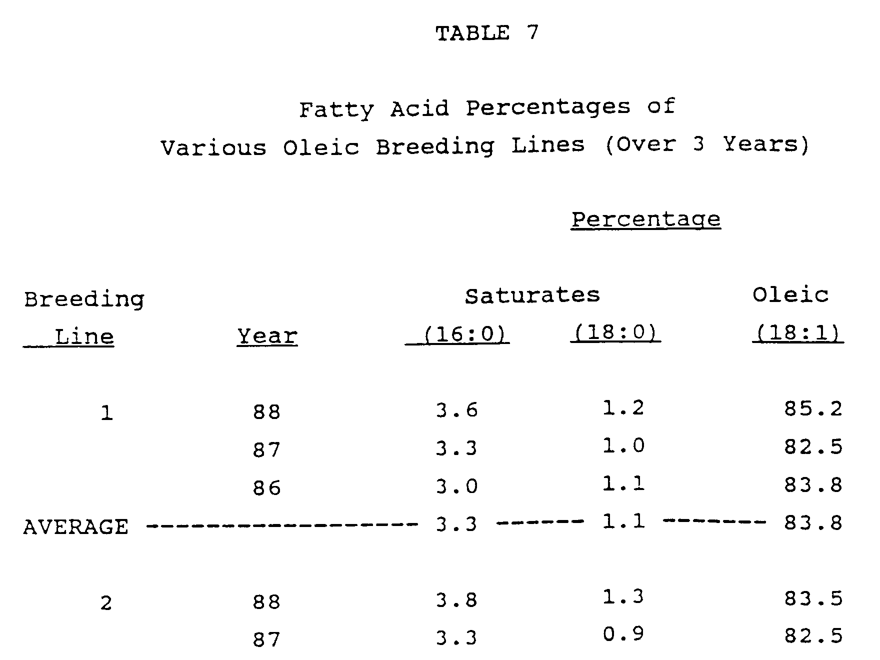

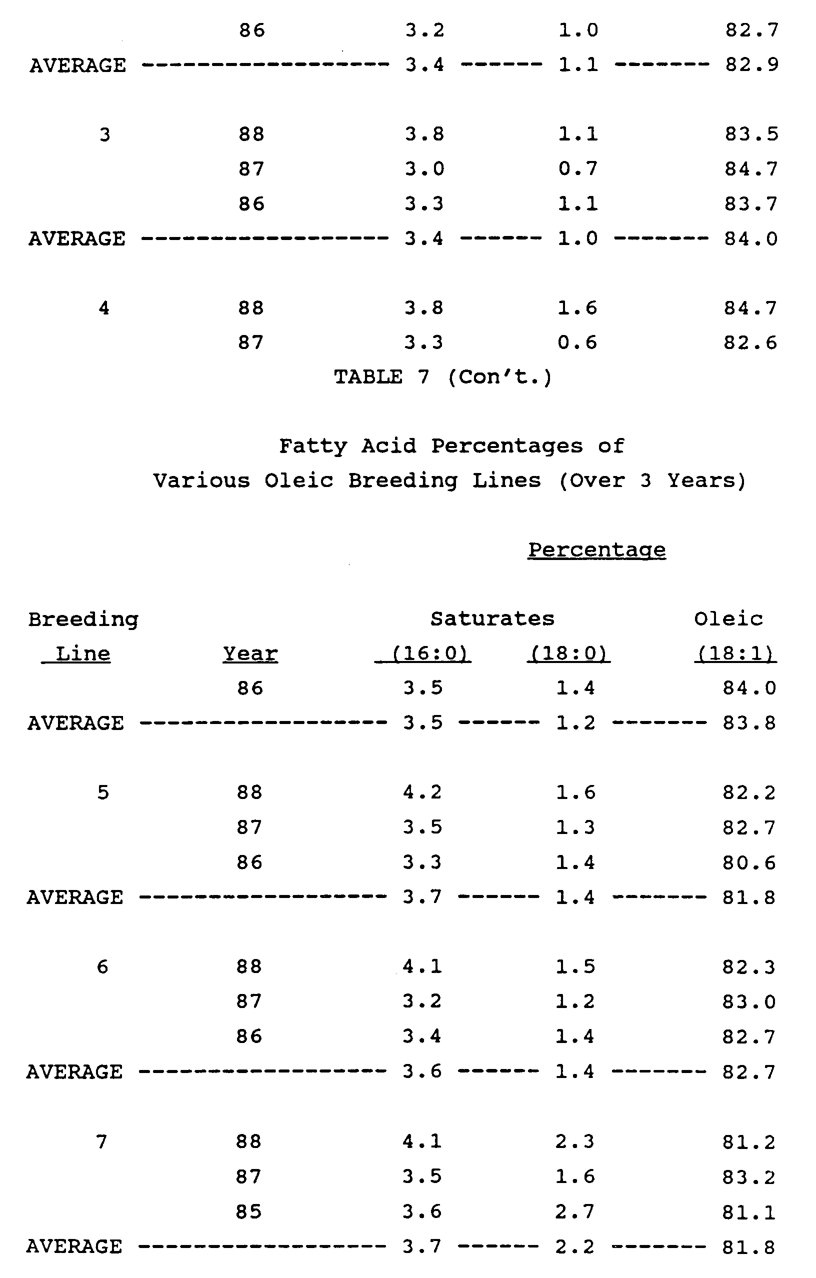

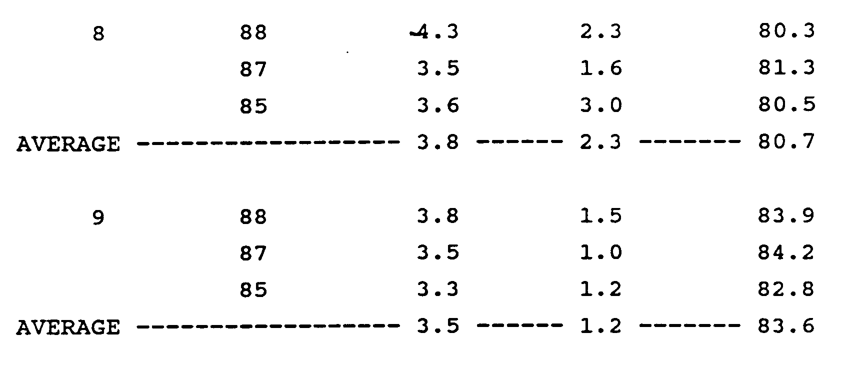

### Example 8

### Breeding Pedigree of Safflower Variety Montola-2000

Montola-2000 was derived from an individual F₆ plant selection from the 1982 cross of a female parent resulting from the multiple cross Frio/Sidney Selection 87-14-6///79AZ9543-1/[S-304/(White US-10/UC-1//Oleic Leed, White S-208//[UC-1/(White US-10/UC-1//Oleic Leed), White S-208]/[Th-5/C. nitidus//ol41-1]] with a male parent resulting from the multiple cross White US-10/UC-1//[S-304/(US-10/UC-1//Oleic leed), White S-208]. From the initial crosses of White flowered US-10/UC-1 and Th-5/ C. nitidus, a total of 19 crosses were made in the breeding of this cultivar. Individual plant selections were made during the F₂, F₃, F₄, F₅ and F₆ generations. Fatty acid determinations were made on seed in the F₆, F₇ and F₈ generations to verify the cultivar produced oil low in saturates and very high in oleic acid (greater than 80%).

The multiple crosses were made in the following scenario.

### Female Parent

- cross 1: - Frio x Sidney Selection 87-14-6 (A)
- White US-10 x UC-1 (B)
- Th-5 x C. nitidus (C)
- cross 2: - (B) progeny x Oleic Leed (D)
- (C) progeny x ol41-1 (E)
- cross 3: - (D) progeny x S-304 (F)
- (D) progeny x UC-1 (G)
- cross 6: - (G) progeny x (E) progeny (H)
- cross 10: - (F) progeny x (H) progeny (I)
- cross 11: - 79AZ9543-1 x (I) progeny (J)
- cross 13: - (A) progeny x (J) progeny (Female Parent)

### Male Parent

- cross 1: - US-10 x UC-1 (K)
- cross 2: - (K) progeny x Oleic Leed (L)
- cross 3: - (L) progeny x S-304 (M)
- cross 4: - White US-10 x UC-1 (N)
- cross 5: - (N) progeny x (M) progeny (Male Parent)

The objective description of variety Montola-2000 is as follows, in accordance with characteristics for a plant variety application.
Maturity - 120 days (Midwest location)
Plant height at maturity - 43 cm
Flower color - Yellow (fresh);
Light orange base (wilted)
Spines on involucral bracts - present at tip and along margins; 2.2 mm in length numbering 15 with a spine index of 33 (class 2 = 21-40)
Heads - 25 mm diameter of primary head;
1-10% seed shattering
Seed - White/white with blotch (4/100); normal hull; 4 mm wide, 7 mm long, 34 g/1000 seeds
Seedling vigor - 8 nodes; 21 cm soil surface to tip
Cold resistance - Rosette: -5°C;
Bolting: 0°C;
Flowering: 5°C
Disease - moderately susceptible to Alternaria leaf spot and Alternaria bud rot
Hull - 44.6%
Protein - 23.0%
Oil - 44.0%
Iodine - 84%
Saturated Acids - 5.5%
Oleic acid - 81.7%
Linoleic acid - 11.4%
The fatty acid profile of variety Montola-2000 was examined in 1987, 1988 and 1989. The results are shown in Table 8.

**TABLE 8**

| Montola-2000 Safflower Fatty Acid Profile 1987 TO 1989 | | | | | |
|---|---|---|---|---|---|
| Seed Source | Year | Palmitic (16:0) | Stearic (18:0) | Oleic (18:1) | Linoleic (18:2) |
| 87b3797-1 | 87 | 3.7 | 1.1 | 82.5 | 11.0 |
| 87b3797-2 | 87 | 3.9 | 1.2 | 81.3 | 12.0 |
| AVERAGE | ------------ | 3.8 ----- | 1.2 ---- | 81.9 ---- | 11.5 |
| 88b5623-1 | 88 | 4.4 | 1.6 | 81.9 | 10.7 |
| 88b5628-1 | 88 | 4.2 | 1.9 | 81.2 | 11.1 |
| 88b5633-1 | 88 | 4.2 | 1.5 | 82.9 | 9.8 |
| 88b5347-1 | 88 | 4.2 | 1.5 | 82.5 | 10.3 |
| 88b5348-1 | 88 | 4.0 | 1.4 | 83.6 | 9.4 |
| 88b5350-1 | 88 | 4.3 | 1.7 | 82.0 | 10.6 |
| AVERAGE | ------------ | 4.2 ----- | 1.6 ---- | 82.4 ---- | 10.3 |
| Breeders Seed-1 | 89 | 4.2 | 1.5 | 80.0 | 12.8 |
| Breeders Seed-1 | 89 | 4.1 | 1.5 | 81.6 | 11.3 |
| Breeders Seed-1 | 89 | 4.2 | 1.3 | 80.6 | 12.7 |
| Breeders Seed-1 | 89 | 4.3 | 1.3 | 81.4 | 12.2 |
| Breeders Seed-1 | 89 | 4.2 | 1.5 | 80.5 | 12.6 |
| AVERAGE | ------------ | 4.2 ----- | 1.4 ---- | 80.8 ---- | 12.3 |

Variety Montola-2000 was compared with several other varieties commonly grown in Montana and North Dakota. The results are shown in Tables 9-19.

**TABLE 9**

| **Relative Test Weights of Safflower Varieties in Montana and North Dakota** | | | | | | |
|---|---|---|---|---|---|---|
| **Variety** | **Williston 1988** | **Sidney 1988** | **Williston 1989** | **Sidney 1989** | **Average Test Weight Lbs/Acre** | **Test Weight in % of MT 3697** |
| Finch | -- | 45.0 | 44.5 | 46.0 | 45.2 | 104.2 |
| MT 3697 | 42.1 | 43.5 | 43.3 | 43.3 | 43.1 | 100.0 |
| S-541 | 40.7 | 41.9 | 42.5 | 43.4 | 42.1 | 97.9 |
| S-317 | 40.7 | 43.0 | 41.4 | 42.8 | 42.0 | 97.5 |
| Girard | 40.9 | 41.2 | 42.0 | 43.5 | 41.9 | 97.3 |
| 87B 3797 | 41.4 | 40.8 | 41.7 | 42.4 | 41.6 | 96.6 |

**TABLE 10**

| **Relative Flowering Date of Safflower Varieties when Grown in the Lower Yellowstone River Valley at the MSU Eastern Agricultural Research Center, Sidney, Montana** | | | |
|---|---|---|---|
| **Variety** | **1988** | **1989** | **Average Flowering Date** |
| Finch | 6 | 25 | 16 |
| Girard | 13 | 27 | 20 |
| S-317 | 11 | 26 | 19 |
| MT 3697 | 10 | 26 | 18 |
| S-541 | 8 | 26 | 17 |
| 87B 3797 | 8 | 25 | 17 |
| C.V. % | 9.4 | 1.2 | |
| L.S.D. 5% | 2.5 | 0.8 | |

**TABLE 11**

| **Relative Yielding Ability of Safflower Varieties when Grown in the Lower Yellowstone River Valley at the MSU Eastern Agricultural Research Center, Sidney, Montana** | | | | |
|---|---|---|---|---|
| **Variety** | **1988** | **1989** | **Average Yield Lbs/Acre** | **Yield in % of MT 3697** |
| S-317 | 4405 | 2573 | 3489 | 120.3 |
| 87B 3797 | 2996 | 2819 | 2908 | 100.3 |
| MT 3697 | 3580 | 2220 | 2900 | 100.0 |
| Girard | 3367 | 2418 | 2893 | 99.7 |
| S-541 | 3195 | 2344 | 2770 | 95.5 |
| Finch | 2946^{*} | 2411 | 2678 | 92.4 |
| C.V.% | 9.8 | 3.1 | | |
| L.S.D. 5% | 76.3 | 20.3 | | |

| | | | | |
|---|---|---|---|---|
| * Relative Performance in Similar Trials | | | | |

**TABLE 12**

| **Relative Disease Rating of Safflower Varieties When Grown in the Lower Yellowstone River Valley at the MSU Eastern Agricultural Research Center, Sidney, Montana** | | |
|---|---|---|
| Variety | 1988 | 1989 |
| 87B 3797 | * | 2.8 |
| S-317 | * | 2.5 |
| Girard | * | 2.2 |
| Finch | * | 1.7 |
| S-541 | * | 2.7 |
| MT 3697 | * | 2.3 |

| | | |
|---|---|---|
| * Disease was not present in 1988. Disease rating in 1989 was principally for Alternaria leaf spot. 5 = most susceptible. | | |

**TABLE 13**

| **Relative Plant Heights of Safflower Varieties When Grown in the Lower Yellowstone River Valley at the MSU Eastern Agricultural Research Center, Sidney, Montana** | | | |
|---|---|---|---|
| **Variety** | **1988** | **1989** | **Average Plant Height Inches** |
| S-317 | 19 | 24 | 22 |
| Finch | 16 | 22 | 19 |
| Girard | 18 | 22 | 20 |
| S-541 | 16 | 23 | 20 |
| MT 3697 | 17 | 21 | 19 |
| 87B 3797 | 14 | 19 | 17 |
| C.V.% | 5.9 | 2.4 | |
| L.S.D. 5% | 2.5 | 1.4 | |

**TABLE 14**

| **Relative Test Weights of Safflower Varieties When Grown in the Lower Yellowstone River Valley at the MSU Eastern Agricultural Research Center, Sidney, Montana** | | | | |
|---|---|---|---|---|
| **Variety** | **1988** | **1989** | **Average Test Weight Lbs/Acre** | **Test Weight in % of MT 3697** |
| Finch | 46.6 | 43.5 | 45.1 | 108.1 |
| 87B 3797 | 41.7 | 41.8 | 41.8 | 100.2 |
| MT 3697 | 42.3 | 41.0 | 41.7 | 100.0 |
| Girard | 42.0 | 41.2 | 41.6 | 99.9 |
| S-541 | 41.5 | 40.2 | 40.9 | 98.1 |
| S-317 | 41.2 | 38.5 | 39.9 | 95.7 |
| C.V. % | 0.8 | 0.7 | | |
| L.S.D. 5% | 0.8 | 0.8 | | |

**TABLE 15**

| **Relative Oil Content of Safflower Varieties When Grown in the Lower Yellowstone River Valley at the MSU Eastern Agricultural Research Center, Sidney, Montana** | | | | |
|---|---|---|---|---|
| **Variety** | **1988** | **1989** | **Average % Oil Content** | **Oil Content in % of MT 3697** |
| 87B 3797 | 46.9 | 46.0 | 46.5 | 104.3 |
| S-541 | 46.3 | 45.5 | 45.9 | 103.0 |
| S-317 | 47.0 | 43.7 | 45.4 | 101.8 |
| MT 3697 | 44.9 | 44.2 | 44.6 | 100.0 |
| Girard | 43.4 | 42.7 | 43.1 | 96.6 |
| Finch | 40.8 | 41.3 | 41.1 | 92.1 |
| C.V.% | 1.1 | 0.5 | | |
| L.S.D. 5% | 1.4 | 0.6 | | |

**TABLE 16**

| **Relative Oil Content of Safflower Varieties in Montana and North Dakota** | | | | | | |
|---|---|---|---|---|---|---|
| **Variety** | **Williston 1988** | **Sidney 1988** | **Williston 1989** | **Sidney 1989** | **Average % Oil Content** | **Oil Content in % of MT 3697** |
| S-541 | 45.2 | 47.9 | 42.9 | 42.8 | 44.7 | 102.8 |
| 87B 3797 | 46.0 | 45.6 | 41.4 | 42.8 | 44.0 | 101.0 |
| S-317 | 44.5 | 46.6 | 40.9 | 43.4 | 43.9 | 100.8 |
| MT 3697 | 45.1 | 46.5 | 40.0 | 42.4 | 43.5 | 100.0 |
| Girard | 42.2 | 42.5 | 39.2 | 39.3 | 40.8 | 93.8 |
| Finch | -- | 42.7 | 37.0 | 38.4 | 39.4 | 93.8 |
| C.V. % | * | 2.9 | * | 1.1 | | |
| L.S.D.5% | * | 3.8 | * | 1.3 | | |

| | | | | | | |
|---|---|---|---|---|---|---|
| * Relative oil content determinations were made on bulk samples from all replications. Oils reported on an oven dry weight basis. Samples dried at 55°C for a minimum of four hours. | | | | | | |

**TABLE 17**

| **Relative Yield Data From Dryland Sites in Montana and North Dakota** | | | | | | |
|---|---|---|---|---|---|---|
| **Variety** | **Williston 1988** | **Sidney 1988** | **Williston 1989** | **Sidney 1989** | **Average Yield Lbs/Acre** | **Yield in % of MT 3697** |
| Finch | -- | * | 619 | 663 | 641 | 144.5 |
| S-317 | 1158 | * | 579 | 708 | 815 | 131.0 |
| S-541 | 944 | * | 508 | 483 | 645 | 103.6 |
| 87B 3797 | 646 | * | 594 | 645 | 628 | 101.0 |
| MT 3697 | 980 | * | 447 | 440 | 622 | 100.0 |
| Girard | 745 | * | 442 | 483 | 557 | 89.5 |
| C.V.% | 28.6 | * | 11.1 | 7.9 | | |
| L.S.D.5% | 25.7 | * | 81.7 | 114.2 | | |

| | | | | | | |
|---|---|---|---|---|---|---|
| * Yields were not taken in 1988 at Sidney, Mt. | | | | | | |

**TABLE 18**

| **Relative Plant Heights of Safflower Varieties in Montana and North Dakota** | | | | | |
|---|---|---|---|---|---|
| **Variety** | **Williston 1988** | **Sidney 1988** | **Williston 1989** | **Sidney 1989** | **Average Plant Height Inches** |
| Finch | -- | 12 | 17 | 18 | 16 |
| S-317 | 11 | 11 | 17 | 19 | 15 |
| Girard | 13 | 12 | 17 | 19 | 15 |
| S-541 | 9 | 10 | 17 | 19 | 14 |
| MT 3697 | 10 | 8 | 16 | 17 | 13 |
| 87B 3797 | 8 | 9 | 15 | 17 | 12 |
| C.V.% | 11.0 | 7.6 | 9.4 | 2.9 | |
| L.S.D. 5% | 2.0 | 2.1 | 2.4 | 1.4 | |

**TABLE 19**

| **Relative Flowering Date of Safflower Varieties in Montana and North Dakota** | | | | | |
|---|---|---|---|---|---|
| **Variety** | **Williston 1988** | **Sidney 1988** | **Williston 1989** | **Sidney 1989** | **Average Flowering Date** |
| Girard | 39 | * | 49 | 23 | 37 |
| S-541 | 37 | * | 49 | 23 | 36 |
| 87B 3797 | 37 | * | 47 | 20 | 35 |
| S-317 | 37 | * | 48 | 21 | 35 |
| MT 3697 | 36 | * | 47 | 21 | 35 |
| Finch | -- | * | 47 | 22 | 35 |
| C.V.% | 2.2 | * | 1.3 | 1.6 | |
| L.S.D. 5% | 1.6 | * | 1.0 | 1.0 | |

| | | | | | |
|---|---|---|---|---|---|
| * No Flowering Notes Taken in 1988. | | | | | |

A total of 2500 seeds of variety Montola-2000 high oleic are concurrently being deposited with the American Type Culture Collection, Rockville, Maryland. The deposit is assigned number 40751. A plant variety protection application is also being concurrently filed.

A total of 2500 seeds of a high linoleic acid oil content are deposited with the American Type Culture Collection and designated as Morlin.

### Example 9

### Breeding Pedigrees of Representative Safflower Varieties

The following high oleic acid safflower varieties were obtained by the described breeding pedigree.

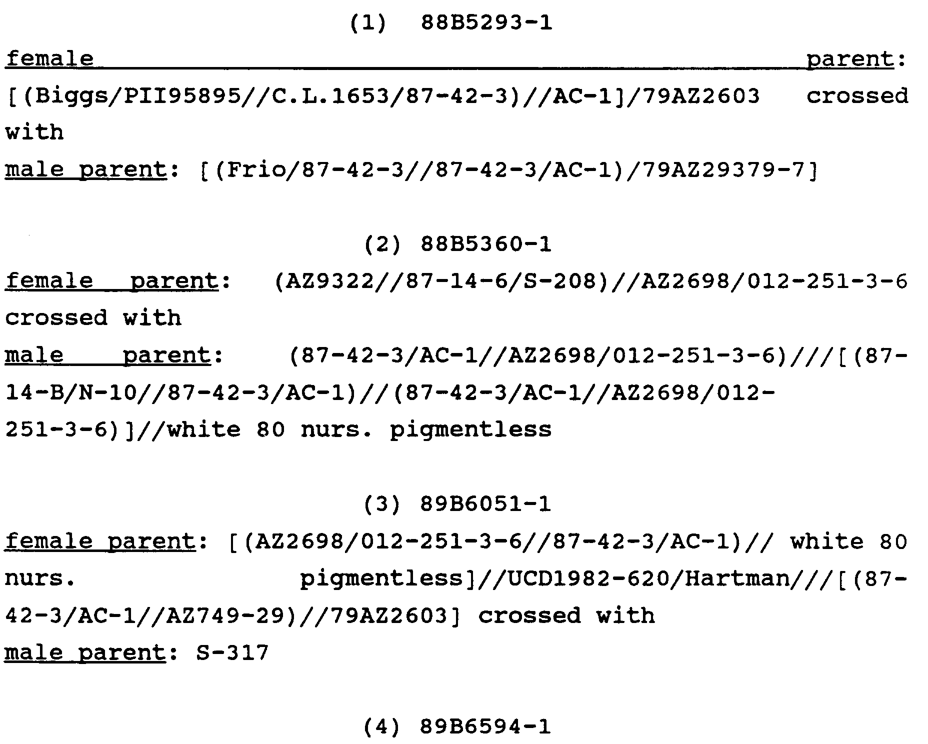

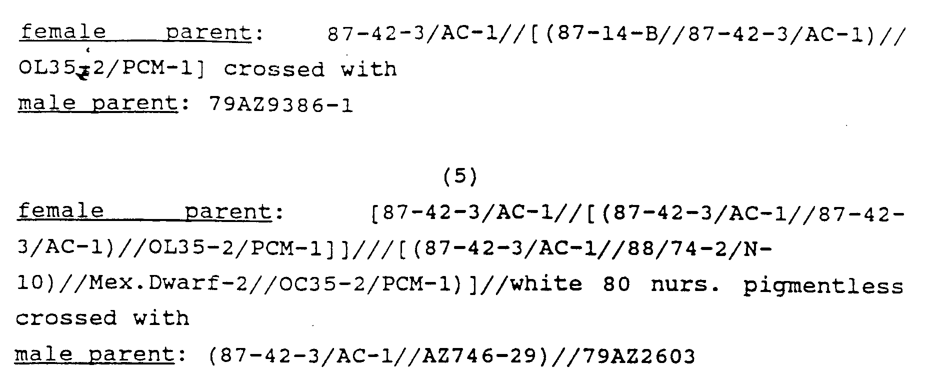

Additional test results obtained on the subject-matter of the invention:

| OLEIC 1990 INCREASE OIL QUALITY ANALYSES | | | | | | | |
|---|---|---|---|---|---|---|---|
| FIELD ENTRY | LAB SAMPLE | OPEN SEED OIL % | TOTAL OPEN SEED | % PALMITIC | % STEARIC | % OLEIC | % LINOLEIC |
| # | # | NMR | grams | 16:0 | 18:0 | 18:1 | 18:2 |
| 90-OL-8111 | 1142 | | | 3.3 | 1.3 | 83.7 | 10.0 |
| 90-OL-8091 | 1140 | | | 3.4 | 1.1 | 83.6 | 10.7 |
| 90-OL-8041 | 1135 | | | 3.4 | 1.2 | 83.2 | 10.5 |
| | | | | AVG 83.5 | | | |

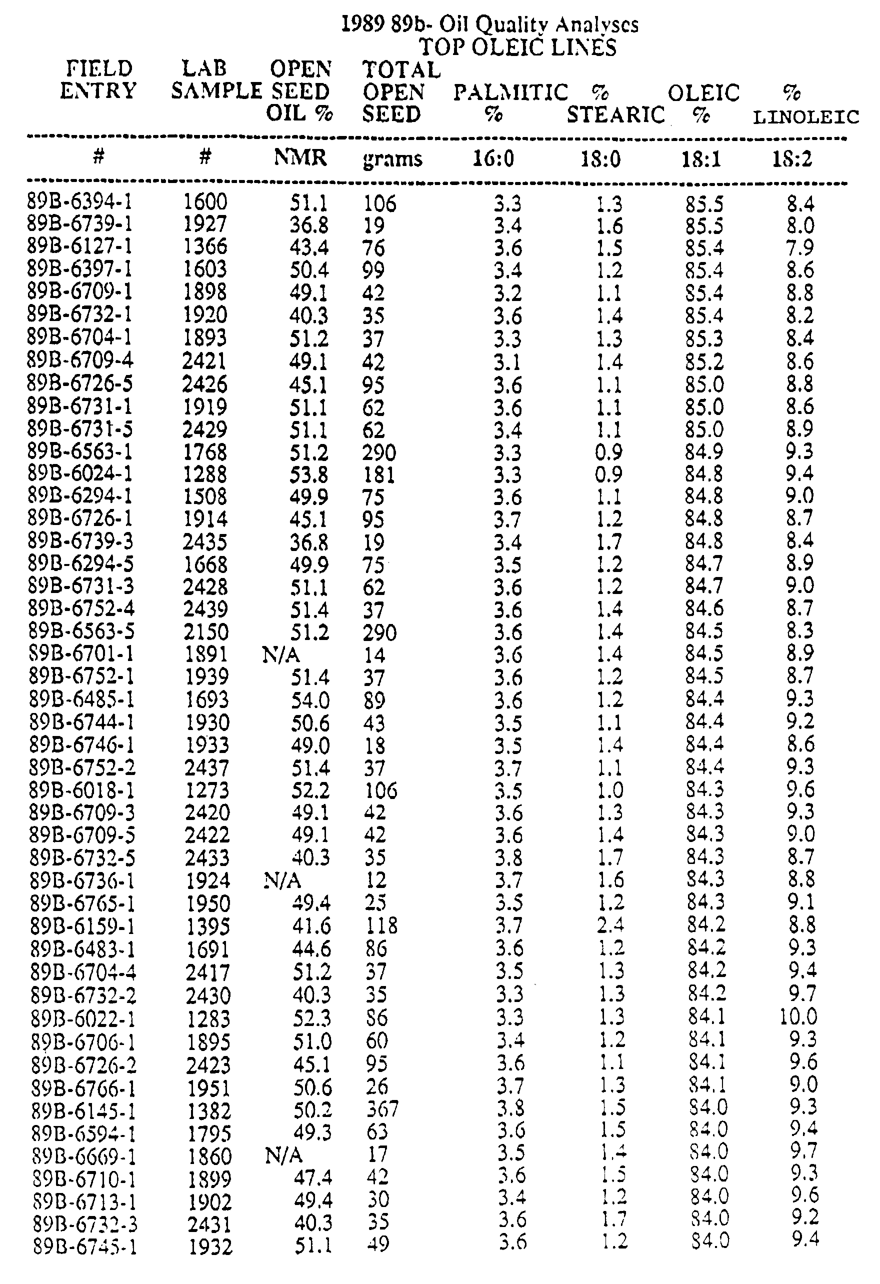

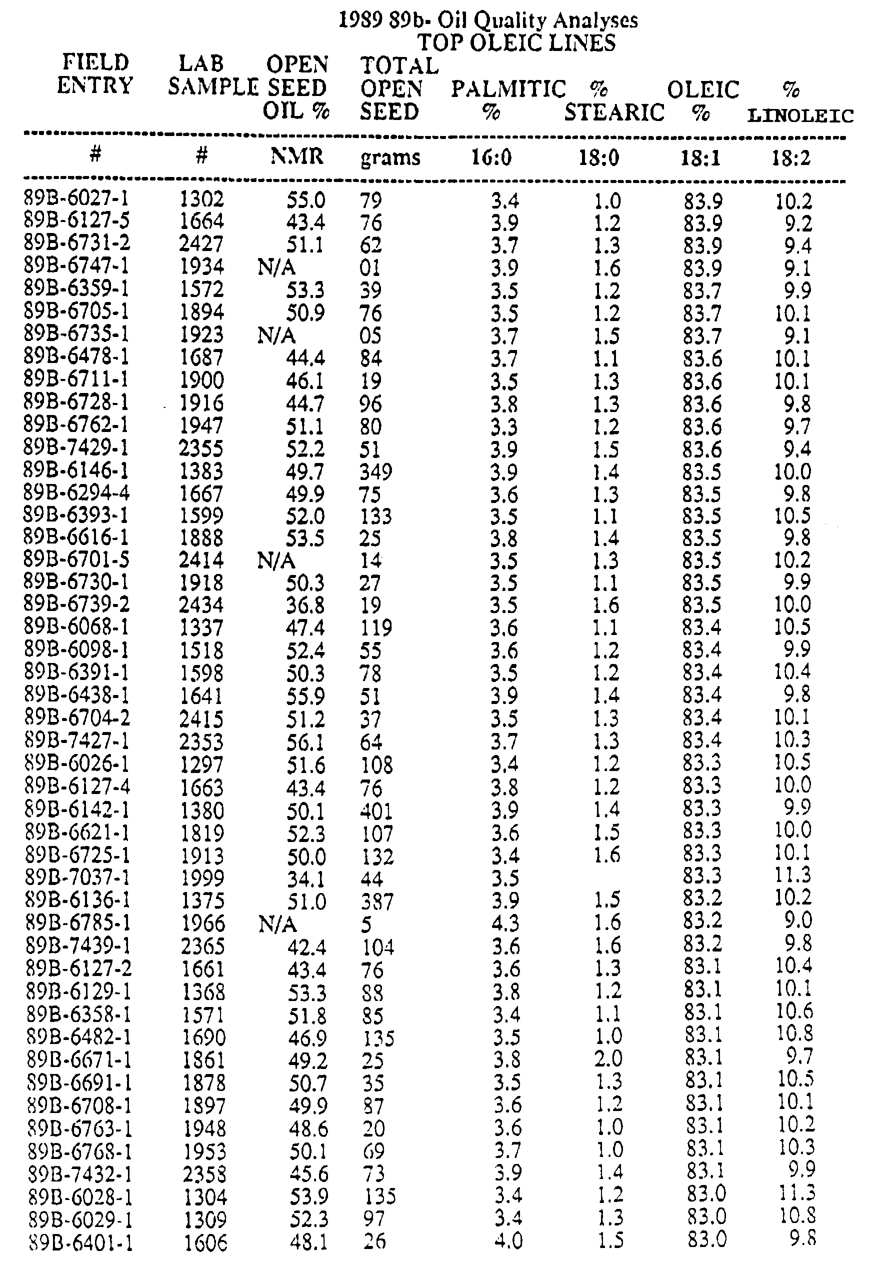

| 1990 Montola-2000 Safflower Single Row Results | | | | | | | |
|---|---|---|---|---|---|---|---|
| FIELD ENTRY | LAB SAMPLE | OPEN SEED OIL % | TOTAL OPEN SEED | % PALMITIC | % STEARIC | % OLEIC | % LINOLEIC |
| # | # | NMR | grams | 16:0 | 18:0 | 18:1 | 18:2 |
| 6720-4 | 3264 | | | 3.8 | 1.4 | 82.8 | 10.0 |
| 6700-5 | 3225 | | | 4.0 | 1.4 | 82.3 | 9.5 |
| 6340-4 | 2954 | | | 3.9 | 1.4 | 82.1 | 9.9 |
| 6380-1 | 1647 | | | 3.8 | 1.6 | 82.1 | 11.1 |
| 6380-1 | 3593 | | | 4.0 | 1.6 | 81.5 | 11.1 |
| 6720-5 | 3265 | | | 4.0 | 1.4 | 81.4 | 11.3 |
| 6340-3 | 2953 | | | 4.4 | 1.7 | 81.4 | 10.9 |
| 6720-3 | 3263 | | | 4.4 | 1.4 | 81.0 | 11.5 |
| 6720-1 | 2033 | | | 3.8 | 1.6 | 80.9 | 12.1 |
| 6340-1 | 1583 | | | 3.9 | 1.2 | 80.6 | 12.9 |
| 6700-1 | 2014 | | | 4.0 | 1.7 | 80.5 | 12.0 |
| 6380-4 | 2966 | | | 4.1 | 1.4 | 80.2 | 12.4 |
| 6380-3 | 2965 | | | 3.9 | 1.2 | 80.1 | 13.1 |
| 6720-2 | 3262 | | | 4.0 | 1.1 | 80.0 | 13.6 |
| 6700-4 | 3224 | | | 4.1 | 1.5 | 80.0 | 12.0 |
| 6900-1 | 2215 | | | 4.2 | 1.5 | 79.9 | 13.0 |
| 6740-1 | 2054 | | | 4.1 | 1.3 | 79.8 | 12.9 |
| 6020-1 | 1259 | | | 4.0 | 1.4 | 79.8 | 13.2 |
| 6300-1 | 1542 | | | 4.0 | 1.3 | 79.6 | 13.2 |
| 6500-1 | 1811 | | | 4.1 | 1.6 | 79.5 | 13.1 |
| 6140-1 | 1381 | | | 4.0 | 1.4 | 79.3 | 13.6 |
| 6340-2 | 2952 | | | 6.2 | 1.7 | 79.3 | 11.3 |
| 6580-1 | 1892 | | | 3.7 | 1.4 | 79.0 | 14.3 |
| | | | | avg 80.6 | | | |

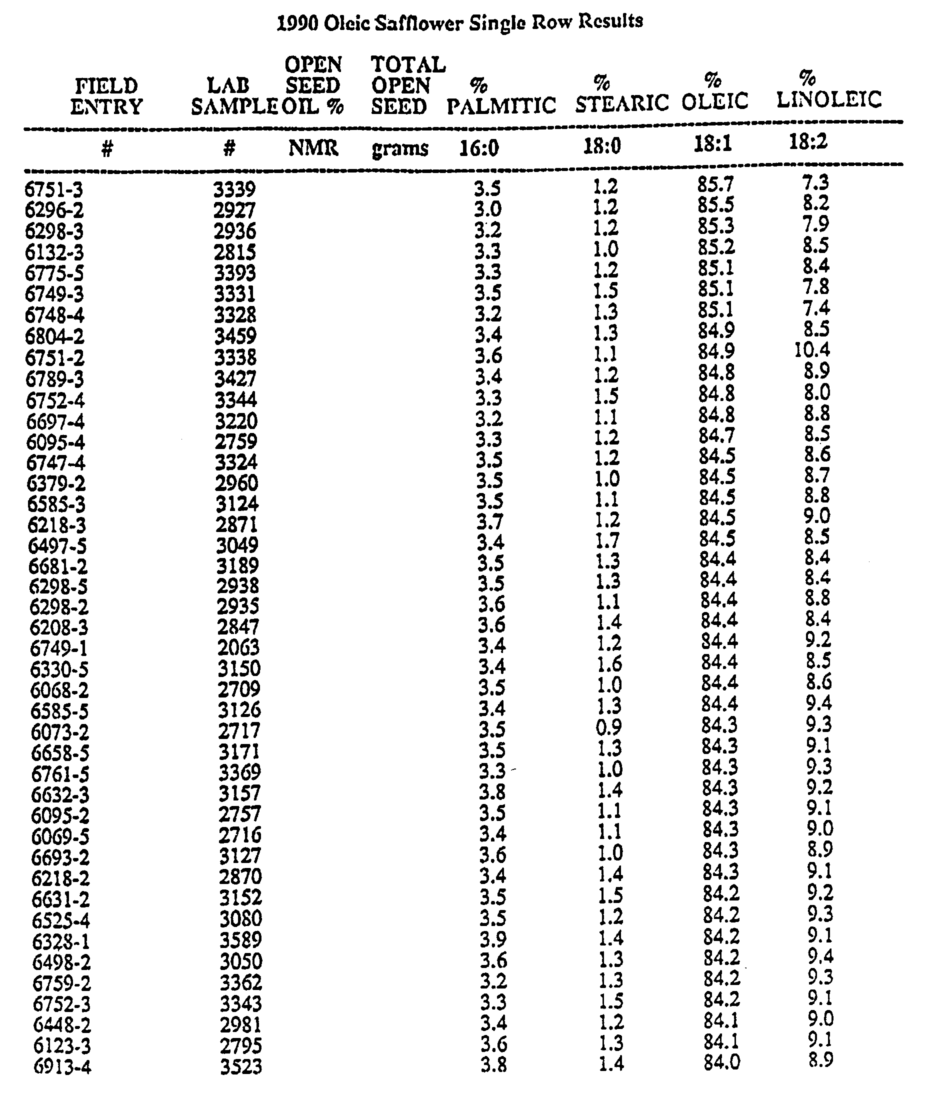

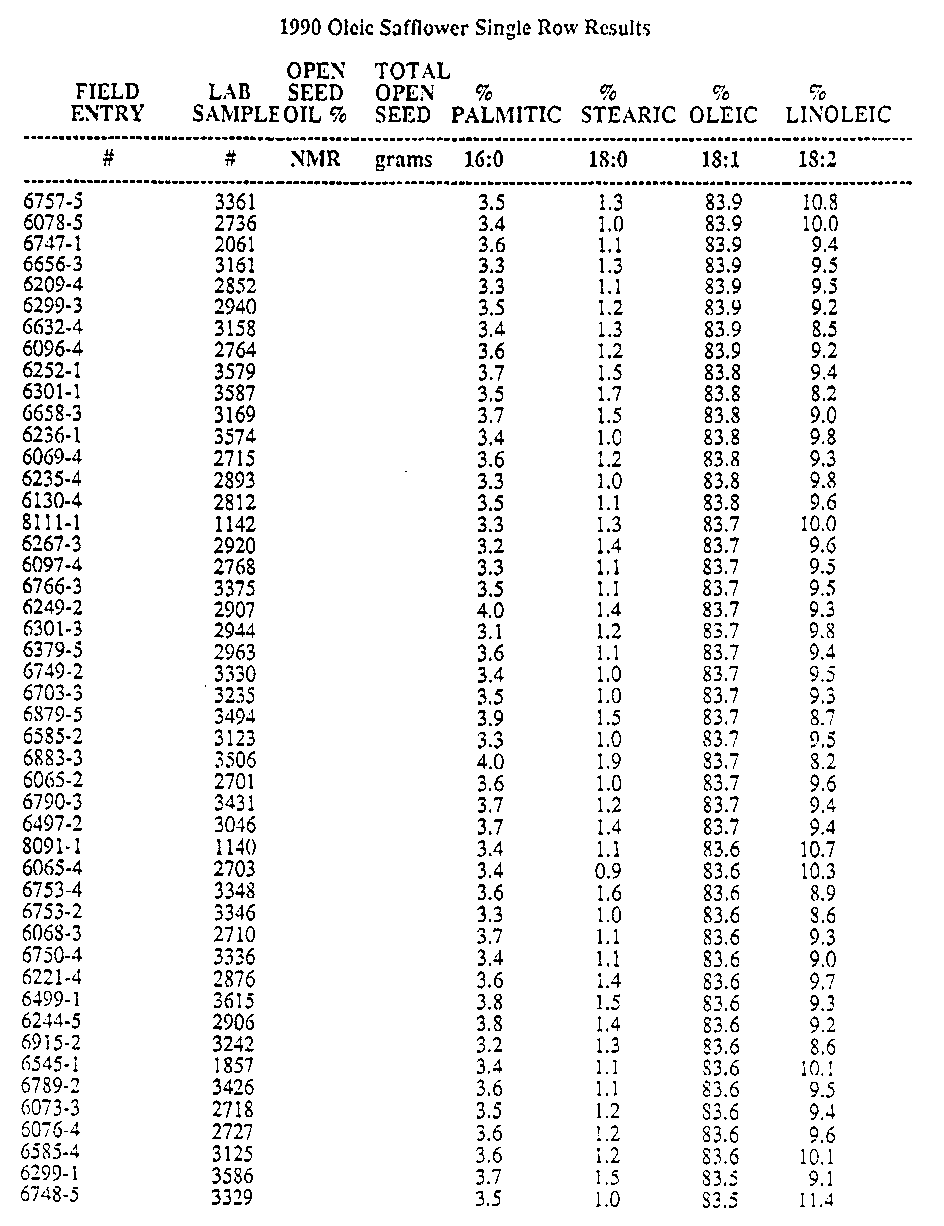

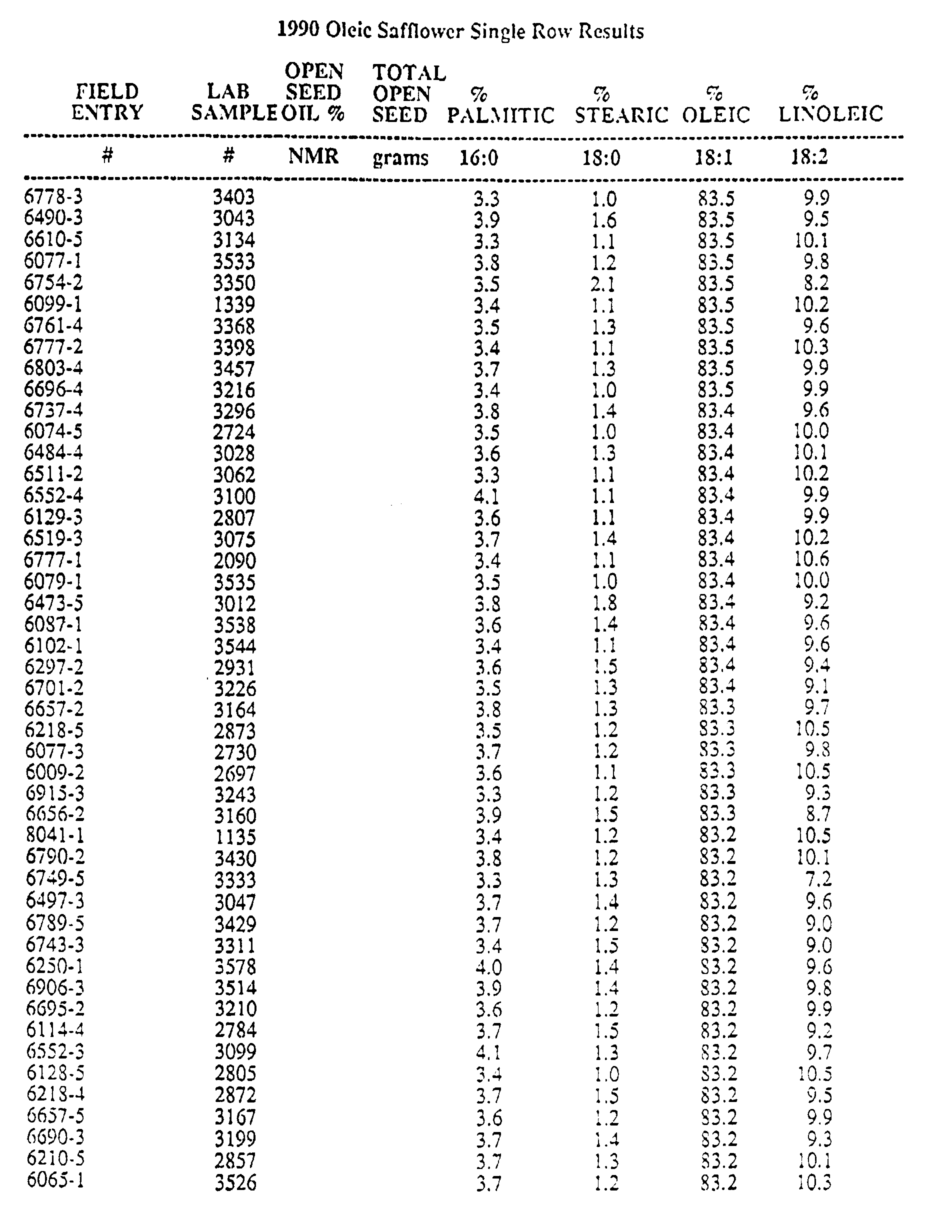

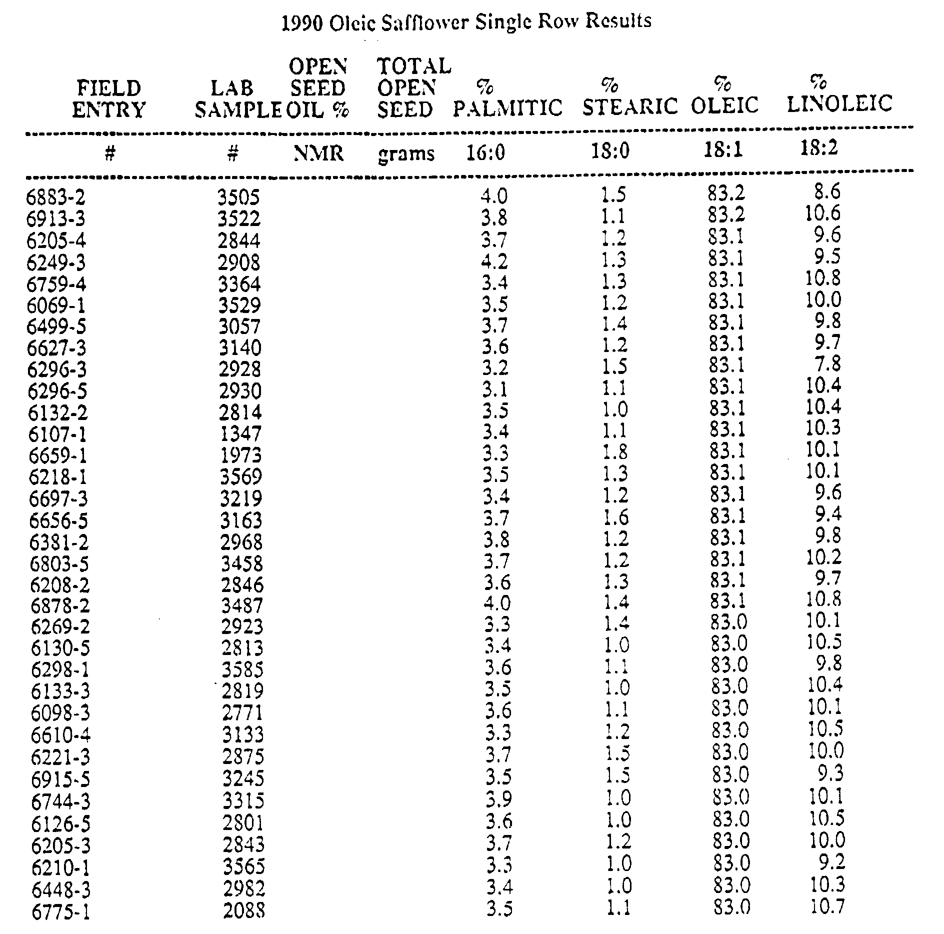

### PROCESSING

The safflower seeds of the present invention may be processed commercially by being ground to a typical size for oil processing. The ground-seed composition is then cooked in a sealed vessel for 1 hour at 130°C. Extraction is then performed in a Butt-extractor, using commercial grade hexane as the solvent for a period of 4 hours. The hexane treated ground composition is then reground and returned to the extractor for an additional 4 hours wherein a second extraction step is conducted using the aforementioned conditions. The solvent is then removed from the miscella, first by distillation to a pot temperature of 75°C and finally into a roto-vac apparatus under reduced pressure of 1.32 KPa (10mm mercury pressure).

The resulting crude extracted oils were each treated with sufficient 16° Baume sodium hydroxide solution to neutralize the free fatty acids plus an additional 1.8% of the sodium hydroxide based on the total oil weight for 5 minutes at 65°C under moderate agitation. The heavy soap phase was separated by centrifugation. Residual soap and impurity removal is accomplished by washing with approximately 15% by volume of water for 5 minutes at 90°C, followed by bleaching the clear oil obtained by centrifugation with 1% by weight acid activated earth at 1.3 to 2.6 KPa (10-20mm) and 95°C for 10 minutes. The activated earth is separated by suction filtration.

The AOM determinations may be conducted pursuant to the American Oil Chemists Society (AOCS) method CD12-57. The AOM content of the high oleic safflower oils described herein is at least about 60 hours, preferably at least 70 hours and most preferably greater than 80 hours in the absence of an antioxidant. If antioxidants are used the AOM values are even greater.

Safflower oil may be identified by a unique sterol. Safflower plants are believed to contain delta-7 stigmasterol which is not believed to be found in other plants. The safflower plants of the present invention are also unique in their ability to fix nitrogen from fertilizer below the level of other plant species. Thus the safflower may be planted on a previously fertilized field and make use of nitrates at lower soil levels. Not only is the fertilizer effectively used but nitrate contamination of ground water is reduced.

## Claims (Claims for the following Contracting State(s): AT, BE, CH, LI, DE, DK, FR, GB, GR, IT, LU, NL, SE)

1. A safflower oil comprising fatty acid, having an oleic acid content of greater than 80% up to 90%, relative to the total fatty acid content of said oil, and having an AOM value of greater than 60 hours.

2. A safflower oil according to claim 1 wherein the oleic acid content is in the range of 83.4% to 90%.

3. A safflower oil according to claim 1 wherein the oleic acid content is at least 81.9%.

4. A safflower oil according to any one of claims 1 to 3 wherein the ratio of the amount of saturated fatty acid relative to the amount of oleic acid in said oil is less than 0.08.

5. A safflower oil according to claim 4 wherein the ratio of saturated fatty acid to oleic acid is less than 0.07.

6. A safflower oil according to any one of claims 1 to 5 wherein the oil is substantially free of free fatty acids.

7. A safflower oil according to any one of claims 1 to 6 wherein the safflower oil is bleached.

8. A safflower oil according to any one of claims 1 to 7 wherein the safflower oil is deodorized.

9. A safflower oil according to any one of claim 1 to 8 wherein the safflower oil is obtained from a homogeneous assemblage of safflower seeds comprising said oil.

## Claims (Claims for the following Contracting State(s): ES)

1. A process for the preparation of a safflower oil comprising fatty acid and having an oleic acid content of greater than 80% up to 90%, relative to the total fatty acid content of said oil, and having an AOM value of greater than 60 hours, which comprises extracting said oil from safflower seeds comprising said oil.

2. A process according to claim 1 wherein the oleic acid content is in the range of 83.4% to 90%.

3. A process according to claim 1 wherein the oleic acid content is at least 81.9%.

4. A process according to any one of claims 1 to 3 wherein the ratio of the amount of saturated fatty acid relative to the amount of oleic acid in said oil is less than 0.08.

5. A process according to claim 4 wherein the ratio of saturated fatty acid to oleic acid is less than 0.07.

6. A process according to any one of claims 1 to 5 wherein the oil is substantially free of free fatty acids.

7. A process according to any one of claims 1 to 6 wherein the safflower oil is bleached.

8. A process according to any one of claims 1 to 7 wherein the safflower oil is deodorized.

9. A process according to any one of claim 1 to 8 wherein the safflower oil is obtained from a homogeneous assemblage of safflower seeds.

## Patentansprüche (Patentansprüche für folgende(n) Vertragsstaat(en): AT, BE, CH, LI, DE, DK, FR, GB, GR, IT, LU, NL, SE)

1. Ein Safloröl, das Fettsäure mit einem Oleinsäuregehalt von größer als 80 % bis zu 90 % bezüglich des Gesamt-Fettsäuregehalts des Öls umfaßt und einen AOM-Wert von größer als 60 Stunden besitzt.

2. Safloröl gemäß Anspruch 1, in welchem der Oleinsäuregehalt im Bereich von 83,4 % bis 90 % liegt.

3. Safloröl gemäß Anspruch 1, in welchem der Ölsäuregehalt wenigstens 81,9 % beträgt.

4. Safloröl gemäß irgendeinem der Ansprüche 1 bis 3, in welchem das Verhältnis der Menge an gesättigter Fettsäure, bezogen auf die Menge an Oleinsäure in dem Öl, weniger als 0,08 beträgt.

5. Safloröl gemäß Anspruch 4, in welchem das Verhältnis von gesättigter Fettsäure zu Oleinsäure weniger als 0,07 beträgt.

6. Safloröl gemäß irgendeinem der Ansprüche 1 bis 5, wobei das Öl im wesentlichen frei von freien Fettsäuren ist.

7. Safloröl gemäß irgendeinem der Ansprüche 1 bis 6, wobei das Safloröl gebleicht ist.

8. Safloröl gemäß irgendeinem der Ansprüche 1 bis 7, wobei das Safloröl geruchsfrei gemacht ist.

9. Safloröl gemäß irgendeinem der Ansprüche 1 bis 8, worbei das Safloröl aus einer homogenen Zusammensetzung von Saflorsamen, welche das Öl umfassen, erhalten wird.

## Patentansprüche (Patentansprüche für folgende(n) Vertragsstaat(en): ES)

1. Ein Verfahren zur Herstellung eines Safloröles, welches Fettsäure mit einem Oleinsäuregehalt von größer als 80 % bis zu 90 % bezüglich des Gesamt-Fettsäuregehalts des Öls umfaßt und einen AOM-Wert von größer als 60 Stunden besitzt, welches ein Extrahieren des Öles aus Saflorsamen, welche das Öl enthalten, umfaßt.

2. Verfahren gemäß Anspruch 1, wobei der Oleinsäuregehalt im Bereich von 83,4 % bis 90 % liegt.

3. Verfahren gemäß Anspruch 1, wobei der Ölsäuregehalt wenigstens 81,9 % beträgt.

4. Verfahren gemäß irgendeinem der Ansprüche 1 bis 3, wobei das Verhältnis der Menge an gesättigter Fettsäure, bezogen auf die Menge an Oleinsäure in dem Öl, weniger als 0,08 beträgt.

5. Verfahren gemäß Anspruch 4, wobei das Verhältnis von gesättigter Fettsäure zu Oleinsäure weniger als 0,07 beträgt.

6. Verfahren gemäß irgendeinem der Ansprüche 1 bis 5, wobei das Öl im wesentlichen frei von freien Fettsäuren ist.

7. Verfahren gemäß irgendeinen der Ansprüche 1 bis 6, wobei das Safloröl gebleicht wird.

8. Verfahren gemäß irgendeinem der Ansprüche 1 bis 7, wobei das Safloröl geruchsfrei gemacht ist.

9. Verfahren gemäß irgendeinem der Ansprüche 1 bis 8, wobei das Safloröl aus einer homogenen Zusammensetzung von Saflorsamen erhalten wird.

## Revendications (Revendications pour l'(les) Etat(s) contractant(s) suivant(s): AT, BE, CH, LI, DE, DK, FR, GB, GR, IT, LU, NL, SE)

1. Huile de carthame comprenant de l'acide gras, ayant une teneur en acide oléique supérieure à 80 % pouvant atteindre 90 %, par rapport à la teneur totale en acide gras de ladite huile, et ayant une valeur AOM supérieure à 60 heures.

2. Huile de carthame selon la revendication 1, où la teneur en acide oléique est dans l'intervalle de 83,4 % à 90 %.

3. Huile de carthame selon la revendication 1, où la teneur en acide oléique est au moins 81,9 %.

4. Huile de carthame selon l'une quelconque des revendications 1 à 3, où le rapport de la quantité d'acide gras saturé à la quantité d'acide oléique dans ladite huile est inférieur à 0,08.

5. Huile de carthame selon la revendication 4, où le rapport d'acide gras saturé à l'acide oléique est inférieur à 0,07.

6. Huile de carthame selon l'une quelconque des revendications 1 à 5, où l'huile est sensiblement exempte d'acides gras libres.

7. Huile de carthame selon l'une quelconque des revendications 1 à 6, où l'huile de carthame est blanchie.

8. Huile de carthame selon l'une quelconque des revendications 1 à 7, où l'huile de carthame est désodorisée.

9. Huile de carthame selon l'une quelconque des revendications 1 à 8, où on obtient l'huile de carthame à partir d'un assemblage homogène de graines de carthame comprenant ladite huile.

## Revendications (Revendications pour l'(les) Etat(s) contractant(s) suivant(s): ES)

1. Procédé de préparation d'huile de carthame comprenant de l'acide gras et ayant une teneur en acide oléique supérieure à 80 % pouvant atteindre 90 %, par rapport à la teneur totale en acide gras de ladite huile et ayant une valeur AOM supérieure à 60 heures, qui comprend l'extraction de ladite huile des graines de carthame comprenant ladite huile.

2. Procédé selon la revendication 1, où la teneur en acide oléique est dans l'intervalle de 83,4 % à 90 %.

3. Procédé selon la revendication 1, où la teneur en acide oléique est au moins 81,9 %.

4. Procédé selon l'une quelconque des revendications 1 à 3, où le rapport de la quantité d'acide gras saturé à la quantité d'acide oléique dans ladite huile est inférieur à 0,08.

5. Procédé selon la revendication 4, où le rapport d'acide gras saturé à l'acide oléique est inférieur à 0,07.

6. Procédé selon l'une quelconque des revendications 1 à 5, où l'huile est sensiblement exempte d'acides gras libres.

7. Procédé selon l'une quelconque des revendications 1 à 6, où l'huile de carthame est blanchie.

8. Procédé selon l'une quelconque des revendications 1 à 7, où l'huile de carthame est désodorisée.

9. Procédé selon l'une quelconque des revendications 1 à 8, où on obtient l'huile de carthame à partir d'un assemblage homogène de graines de carthame.
